# EUROPEAN PATENT SPECIFICATION

(11) **EP 3 454 781 B1**
(45) Date of publication and mention of the grant of the patent: **26.03.2025**
(21) Application number: 17795739.6
(22) Date of filing: 09.05.2017
(51) Int. Cl.: A61F 2/02, A61L 15/58, A61L 31/04, A61L 31/12

(54) **MESH-BASED IN SITU CROSS-LINKABLE COMPOSITIONS**
NETZBASIERTE IN-SITU VERNETZBARE ZUSAMMENSETZUNGEN
COMPOSITIONS RÉTICULABLES IN SITU À BASE D'UNE STRUCTURE MAILLÉE

(30) Priority: 09.05.2016 US 201662333521 P
(43) Date of publication of application: 20.03.2019
(73) Proprietor: Bard Shannon Limited, Humacao 00791 (PR)
(72) Inventor: PREISS-BLOOM, Orahn, 30900 Zichron Yaakov (IL); TOMER, Guy, 71701 Modiin (IL); HADID, Amir, 3055155 Binyamina (IL); POLAKEWICZ, Alon, 3708450 Pardes Hanna (IL); MAIZLER, Ariel, 3822153 Hadera (IL); KRAMARENKO, Denis, 3385111 Haifa (IL); GODER, Daniella, 6266817 Tel Aviv (IL); YOSUFOV, Danny, 3849269 Hadera (IL)
(74) Representative: FRKelly
(86) International application number: PCT/IL2017/050512
(87) International publication number: WO 2017/195198

(56) References cited:
- WO-A2-2009/153748
- WO-A2-2009/153750
- WO-A2-2009/153750
- US-A1- 2013 131 701
- US-A1- 2013 131 701
- US-A1- 2015 133 377

## Description

### FIELD OF THE INVENTION

The present invention relates to coated surgical mesh whereas the coating is based on cross-linked compositions comprising a cross-linkable protein and an enzyme which induces cross-linking of the cross-linkable protein, applied with a backing layer.

### BACKGROUND OF THE INVENTION

Biomaterials that can form gels *in situ* are useful for a variety of applications. In many cases, *in situ* gel-forming materials are used as injectable matrices for controlled drug delivery or injectable scaffolds for tissue engineering. (Gutowska A, Jeong B, Jasionowski M. Anat Rec 2001, 263, 342-349. Silva EA, Mooney DJ. J Thromb Haemost 2007, 5, 590-8. Mahoney MJ, Anseth KS. J Biomed Mater Res A 2007, 81, 269-78.) *In situ* gel-forming materials can also serve as adhesives to bond tissue or seal leaks (either gas or fluid) in a physiological environment.

Interest in soft tissue adhesives is growing because of the desire to replace or supplement sutures for wound closure (Glickman M, Gheissari A, Money S, Martin J, Ballard J. Arch Surg 2002, 137, 326-31; discussion 332. Pursifull NF, Morey AF. Curr Opin Urol 2007, 17, 396-401.), the trends toward less invasive and cosmetic surgeries (Tissue Adhesives in Clinical Medicine; 2nd ed.; Quinn, J. V., Ed.; BC Decker: Hamilton, Ontario Canada, 2005*.* Tissue Glue in Cosmetic Surgery; Saltz, R.; Toriumi, D. M., Eds.; Quality Medical Publishing, Inc.,: St. Louis, Missouri, USA 2004.), and the need for emergency hemostasis (Pusateri AE, Holcomb JB, Kheirabadi BS, Alam HB, Wade CE, Ryan KL. Journal of Trauma-Injury Infection and Critical Care 2006, 60, 674-682. Acheson EM, Kheirabadi BS, Deguzman R, Dick EJ, Holcomb JB. Journal of Trauma-Injury Infection and Critical Care 2005, 59, 865-874. Kheirabadi BS, Acheson EM, Deguzman R, Sondeen JL, Ryan KL, Delgado A, Dick EJ, Holcomb JB. Journal of Trauma-Injury Infection and Critical Care 2005, 59, 25-34.)

*In situ* gel formation can be initiated by a variety of approaches. Chemical approaches to gel formation include the initiation of polymerization either by contact, as in cyanoacrylates, or external stimuli such as photo-initiation. Also, gel formation can be achieved by chemically crosslinking pre-formed polymers using either low molecular weight crosslinkers such as glutaraldehyde or carbodiimide (Otani Y, Tabata Y, Ikada Y. Ann Thorac Surg 1999, 67, 922-6. Sung HW, Huang DM, Chang WH, Huang RN, Hsu JC. J Biomed Mater Res 1999, 46, 520-30. Otani, Y.; Tabata, Y.; Ikada, Y. Biomaterials 1998, 19, 2167-73. Lim, D. W.; Nettles, D. L.; Setton, L. A.; Chilkoti, A. Biomacromolecules 2008, 9, 222-30.), or activated substituents on the polymer (Iwata, H.; Matsuda, S.; Mitsuhashi, K.; Itoh, E.; Ikada, Y. Biomaterials 1998, 19, 1869-76).

In addition to chemical approaches, gel formation can be achieved through physical means using self-assembling peptides (Ellis-Behnke RG, Liang YX, Tay DK, Kau PW, Schneider GE, Zhang S, Wu W, So KF. Nanomedicine 2006, 2, 207-15. Haines-Butterick L, Rajagopal K, Branco M, Salick D, Rughani R, Pilarz M, Lamm MS, Pochan DJ, Schneider JP. Proc Natl Acad Sci U S A 2007, 104, 7791-6. Ulijn RV, Smith AM. Chem Soc Rev 2008, 37, 664-75).

Finally, biological approaches to initiate gel formation have been investigated based on the crosslinking components from marine adhesives, such as mussel glue (Strausberg RL, Link RP. Trends Biotechnol 1990, 8, 53-7), or blood coagulation, as in fibrin sealants (Jackson MR. Am J Surg 2001, 182, 1S-7S. Spotnitz WD. Am J Surg 2001, 182, 8S-14S Buchta C, Hedrich HC, Macher M, Hocker P, Redl H. Biomaterials 2005, 26, 6233-41.27-30).

A variety of biomimetic approaches have also been considered for *in situ* gel formation. In these approaches, polymer crosslinking and gel formation are modeled after one of the crosslinking operations found in biology. The biological model that has probably attracted the most technological interest is the mussel glue that sets under moist conditions (Silverman HG, Roberto FF. Mar Biotechnol (NY) 2007, 9, 661-81. Deacon MP, Davis SS, Waite JH, Harding SE. Biochemistry 1998, 37, 14108-12.). Cross-linking of the mussel glue is initiated by the enzymatic conversion of phenolic (i.e., dopa) residues of the adhesive protein into reactive quinone residues that can undergo subsequent inter-protein crosslinking reactions (Burzio LA, Waite JH. Biochemistry 2000, 39, 11147-53. McDowell LM, Burzio LA, Waite JH, Schaefer JJ. Biol Chem 1999, 274,20293-5). A second biological cross-linking operation that has served as a technological model is the transglutaminase-catalyzed reactions that occur during blood coagulation (Ehrbar M, Rizzi SC, Hlushchuk R, Djonov V, Zisch AH, Hubbell JA, Weber FE, Lutolf MP. Biomaterials 2007, 28, 3856-66). Biomimetic approaches for *in situ* gel formation have investigated the use of Factor XIIIa or other tissue transglutaminases (Sperinde J, Griffith L. Macromolecules 2000, 33, 5476-5480. Sanborn TJ, Messersmith PB, Barron AE. Biomaterials 2002, 23, 2703-10).

One biomimetic approach for *in situ* gel formation of particular interest is the crosslinking of gelatin by a calcium independent microbial transglutaminase (mTG). mTG catalyzes an analogous crosslinking reaction as Factor XIIIa but the microbial enzyme requires neither thrombin nor calcium for activity. Initial studies with mTG were targeted to applications in the food industry (Babin H, Dickinson E. Food Hydrocolloids 2001, 15, 271-276. Motoki M, Seguro K. Trends in Food Science & Technology 1998, 9, 204-210.), while later studies considered potential medical applications. Previous *in vitro* studies have shown that mTG can crosslink gelatin to form a gel within minutes, the gelatin-mTG adhesive can bond with moist or wet tissue, and the adhesive strength is comparable to, or better than, fibrin-based sealants (Chen TH, Payne GF, et al. Biomaterials 2003, 24, 2831-2841. McDermott MK, Payne GF, et al. Biomacromolecules 2004, 5, 1270-1279. Chen T, Payne GF, et al. J Biomed Mater Res B Appl Biomater 2006, 77, 416-22.).

Application of various biocompatible materials as discussed above has also been augmented with a surgical mesh and a backing layer.

Surgical meshes are porous sheet materials which may be woven or spun from a variety of organic and synthetic materials. The materials from which surgical meshes are made must be biocompatible, chemically and physically inert, non-carcinogenic, mechanically strong and easily fabricated and sterilized.

In many surgical procedures, it is desirable that a surgical mesh becomes incorporated into the tissues surrounding a surgical site. Such surgical procedures include the repair of anatomical defects of the abdominal wall, diaphragm and chest wall, correction of defects in the genitourinary system and repair traumatically damaged organs such as the spleen, liver or kidney. Another example of such a surgical procedure is the reinforcement of a herniation. In the repair of a hernia, a surgical mesh of appropriate size and shape is placed over the hernia and secured in place using any suitable connecting means. As the tissues surrounding the surgical site heal, granulation tissues growing at and around the surgical site begin to produce an extracellular matrix which, in a process called fibrosis, infiltrates and attaches to the material of the surgical mesh secured over the surgical site. Incorporation of the surgical mesh into the surgical site by the extracellular matrix strengthens the tissues at the surgical site and helps prevent recurring injury.

Methods of mesh fixation that are commonly employed are placement of metal fixation devices (tacks) combined with either absorbable or non-absorbable transabdominal sutures and the insertion of two circles of tacks without the use of sutures (the double-crown technique). The fixation of mesh to the abdominal wall using tacks or stitches is recognized as a casual factor in postoperative pain by causing direct nerve and tissue injury and it has been found that patients undergoing laparoscopic ventral and incisional hernia repair tend to have more pain in the early postoperative period than after any other minimally invasive surgery(Wassenaar, E., et al., Mesh-fixation method and pain and quality of life after laparoscopic ventral or incisional hernia repair: a randomized trial of three fixation techniques. Surgical endoscopy, 2010. 24(6): p. 1296-1302. Champault, G., et al., A self-adhering mesh for inguinal hernia repair: preliminary results of a prospective, multicenter study. Hernia, 2011. 15(6): p. 635-641). Estimated rates of chronic pain vary considerably from 0 to 53%. Up to one third of patients will complain of some degree of pain one year after surgery and in 3-4% of patients, this pain will be severe and disabling, significantly affecting the patients' quality of life (Champault, G., et al., A self-adhering mesh for inguinal hernia repair: preliminary results of a prospective, multicenter study. Hernia, 2011. 15(6): p. 635-641). In the case of mesh repair of inguinal hernias, an increasing number of clinicians and researchers now consider postoperative pain the most important adverse effect of laparoscopic ventral and incisional hernia repair surgeries surgeries (Wassenaar, E., et al., Mesh-fixation method and pain and quality of life after laparoscopic ventral or incisional hernia repair: a randomized trial of three fixation techniques. Surgical endoscopy, 2010. 24(6): p. 1296-1302.). Recent research has been focused on finding new and less pain-inducing mesh fixation techniques, including the use of surgical adhesives. Olmi et al. observed a low rate of postoperative pain in a series of 40 patients in which fibrin glue was used to fix the mesh during laparoscopic repair of small and medium-sized abdominal wall defects (Wassenaar, E., et al., Mesh-fixation method and pain and quality of life after laparoscopic ventral or incisional hernia repair: a randomized trial of three fixation techniques. Surgical endoscopy, 2010. 24(6): p. 1296-1302. Olmi, S., et al., Use of fibrin glue (Tissucol®) in laparoscopic repair of abdominal wall defects: preliminary experience. Surgical endoscopy, 2007. 21(3): p. 409-413).

Conventional tissue adhesives are generally not suitable for a wide range of adhesive applications. While a number of surgical adhesives are currently used in the surgical arena, no existing commercially available product is both safe to use and sufficiently strong to provide the mechanical and biological support necessary to fixate hernia mesh. Furthermore, no existing commercially available product can provide sufficient adhesive strength to strongly adhere implantable medical devices to tissue sites while allowing for rapid tissue ingrowth.

For example, cyanoacrylate based adhesives have been used for topical wound closure, but the release of toxic degradation products limits their use for internal applications. In any case, such adhesives do not allow for tissue integration. Fibrin-based adhesives are slow curing, have poor mechanical strength and pose a risk of viral infection. There have been advances in the field of protein-based tissue adhesives such as an albumin based adhesive, crosslinked with a carbodiimide with the addition of a polyamine, specifically poly(lysine) or chitosan, or a polycarboxylate, specifically citric acid or poly(acrylic acid), to increase the rate of crosslinking are described by Wilkie et al. (U .S. Patent Application Publication No. 2002/0022588) and Tammishetti et al. (WO 99/66964), but the use of carbodiimides in the adhesive composition causes a toxicity problem. The toxicity problem is exacerbated by the use of a toxic polyamine such as poly(lysine). Otani et al. describe a tissue adhesive prepared by crosslinking gelatin and poly(L-glutamic acid) with a water-soluble carbodiimide (Otani, Y., Y. Tabata, and Y. Ikada, A new biological glue from gelatin and poly (L-glutamic acid). Journal of biomedical materials research, 1996. 31(2): p. 157-166). Although the adhesive is less toxic than the albumin-poly(lysine) adhesive described above, it lacks adhesive strength.

There are currently two self fixating hernia meshes on the market. Adhesix^{®} (formely Cousin Biotech, now Bard-Davol) is a mesh coated with a layer of polyvinyl pyrolidonne (PVP) and PEG. It becomes tacky when wetted. Adhesix^{®} meshes were shown to dislocate 50 % of the time in a rat hernia online model after 14 days and 90 days (Gruber-Blum S., et al. (2014) A comparison of Progrip(®) and Adhesix (®) self-adhering hernia meshes in an onlay model in the rat, Hernia 18:761-9). ProGrip^{™} (Medtronic) is a self fixation hernia mesh. It relies on the mechanical principle of micro grips made of polylactic acid (PLA). This product cannot be used in intraperitoneal hernia repair due to the lack of a visceral adhesion protection layer and poor fixation to the peritoneum tissue.

Another issue that has been found to be problematic with surgical meshes is the formation of adhesions to the mesh. While experimenting with Pro-Tack, LeBlanc et al. have observed a "rollover" of the edge of the mesh onto itself at the site of placement of the fixation device. In their study, they determined that this appeared to enhance the instance of adhesions to the exposed edge of the prosthesis (LeBlanc, K., et al., Comparison of adhesion formation associated with Pro-Tack (US Surgical) versus a new mesh fixation device, Salute (ONUX Medical). Surgical Endoscopy and Other Interventional Techniques, 2003. 17(9): p. 1409-1417). More research has found that there is a significantly higher percentage of adhesions to bare polypropylene mesh than to a polypropylene mesh coated on one side with an anti-adhesion barrier (Borrazzo, E., et al., Effect of prosthetic material on adhesion formation after laparoscopic ventral hernia repair in a porcine model. Hernia, 2004. 8(2): p. 108-112).

United States Patent Application No. 20100305589 describes a textile implant that is coated with a bioadhesive. The bioadhesive includes various synthetic polymers and a plasticizer, but not a protein such as gelatin for example. United States Patent Application No 20120197415 describes an implant but does not relate to enzymatic cross-linking of gelatin. United States Patent Application No 20130158571 also does not relate to gelatin. United States Patent Application No 20130131701 describes a patch comprising an implantable surgical mesh, a cross-linkable protein matrix, gelatin, and a protein cross-linking enzyme in contact with said matrix for cross-linking said cross-linkable protein.

### SUMMARY OF THE INVENTION

The background art does not teach or suggest a self-adhering mesh that does not require additional means of fixation, and yet at the same time prevents any "rollover" and exposure of bare mesh, resulting in the minimization of adhesions.

The background art does not teach or suggest a mesh-based composition which is both safe to use and sufficiently strong to provide the mechanical and biological support necessary to, for example, fixate hernia mesh. Furthermore, no existing commercially available product can provide sufficient adhesive strength to strongly adhere implantable medical devices to tissue sites while allowing for rapid tissue ingrowth.

The present invention provides a mesh-based composition comprising a mesh-based composition comprising a mesh and a coating, wherein said coating is a foamed composition comprising one or more cross-linkable proteins or polypeptides and one or more cross-linking materials, wherein at least a portion of said mesh is coated with said coating such that the mesh-based composition comprises two sections, a mesh-adhesive section which is composed of a mesh enclosed within said coating and a coating-only section which contains said coating alone, without mesh within it, wherein the coating-only section surrounds the mesh-adhesive section, and wherein the coated mesh is self-adhering, requires no additional fixation, and is capable of minimizing tissue adhesions upon application..

The mesh-based composition comprises a self-adhering surgical mesh which requires no additional fixation means while at the same time minimizing adhesions. The mesh comprises a composite mesh featuring a foamed composition of the cross-linkable protein and a non-toxic material that induces cross-linking of the cross-linkable material. The composite mesh may optionally be in a sheet-like form. The cross-linkable protein or polypeptide may optionally comprise gelatin which may optionally be foamed, optionally also present in a layer.

According to some demonstrative embodiments, the gelatin layer described hereinabove may optionally be foamed, for example, by mixing the gelatin solution with pressurized air and/or other gas prior to drying. In some embodiments, the gelatin foam may be in a density range of 1 to 100 mg/cm³ and preferably in the range of 1 to 50 mg/cm³.

According to at least some embodiments, preferably the composite mesh features an incorporated hernia mesh. The structure features two sections, one of which is composed of a mesh enclosed within the adhesive composition and another which contains the adhesive composition alone, without mesh within it. This design allows for a self-fixating adhesion minimizing hernia mesh device, preventing the mesh from shifting, migrating, rolling up its edges, or changing its position without the use of sutures, staples and other additional means of fixation.

Said gelatin comprises foamed gelatin. Optionally said foamed gelatin comprises dried or lyophilized foamed gelatin solution. Optionally said enzyme is present in an enzymatic layer and wherein said gelatin is positioned in one or more of the following locations: within said product, on said enzymatic layer, in said enzymatic layer, on said reinforcing back layer, in said reinforcing back layer, or between said an enzymatic layer and said reinforcing back layer.

Optionally said gelatin is foamed gelatin and wherein prior to foaming, the concentration of the gelatin solution is between 0.1% and 30% w/w. Optionally prior to foaming, the concentration of the gelatin solution is between 1% and 20% w/w. Optionally prior to foaming, the concentration of the gelatin solution is between 5% and 15% w/w.

Optionally said cross-linkable protein is present in a protein matrix, wherein said dry matrix has a density in a range of from 1 to 100 mg/cm³. Optionally said density is in a range of from 1 to 50 mg/cm³.

Optionally said foamed gelatin is produced according to a method selected from the group consisting of a batch mixing process, a continuous mixing process, a chemical foaming process, a Venturi foaming process or freeze drying.

Optionally said protein comprises gelatin and the cross-linking agent, such as an enzyme, comprises transglutaminase (TG). Optionally the gelatin is incorporated into a gelatin matrix with said transglutaminase such that one or more of the following occur: a majority of enzyme activity is preserved throughout a process of preparation; enzyme is equally distributed across the gelatin matrix surface; and/or enzyme is embedded into the depth of the gelatin matrix (gradient or equal distribution). Optionally said transglutaminase is incorporated into said gelatin matrix according to one or more of mixing before drying said matrix or after drying said matrix, optionally wherein said matrix is dried to comprise no more than 10% moisture content. Optionally a density of said dry matrix is in a range of 1-100 mg/cm³, or transglutaminase is present at a concentration of from 0.05 to 2 mg transglutaminase/cm³ gelatin matrix. Preferably, the transglutaminase composition has a specific activity level (enzyme units/protein content) of about at least 1 U/mg. Most preferably, the transglutaminase has a specific activity level of at least about 5 U/mg.

Optionally and preferably, the activity level of the transglutaminase in the gelatin-transglutaminase composition is from about 1 to about 500 U/g of gelatin. More preferably, the activity level is from about 5 to about 120 U/g of gelatin.

According to some embodiments of the present invention, there is provided a composition comprising a cross-linkable protein or polypeptide, with the proviso that said protein or polypeptide is not fibrin or fibrinogen, and a cross-linking agent, optionally an enzyme.

Optionally and preferably, for any composition described herein, the enzyme comprises one or more of transglutaminase or a multi-copper oxidase.

More preferably said transglutaminase comprises microbial transglutaminase.

According to some embodiments of the present invention, there is provided a composition comprising a cross-linkable protein or polypeptide, with the proviso that said protein or polypeptide is not fibrin or fibrinogen, an enzyme which induces cross-linking of said cross-linkable protein, a metal ion and a denaturing agent.

Optionally the cross-linkable protein or polypeptide comprises gelatin. Preferably, said gelatin is at least 250 bloom.

More preferably said metal ion comprises calcium as any pharmaceutically compatible salt. Most preferably, said calcium salt comprises one or more of calcium chloride or calcium hydroxide. Optionally and most preferably, said calcium is present in an amount of up to 1M.

According to some embodiments of the present invention, there is provided a composition comprising gelatin, transglutaminase and a calcium crosslinkable alginate matrix. Optionally, exposing said composition to a calcium ion rich moist environment causes cross-linking of the alginate to occur. Without wishing to be limited by a single hypothesis, such cross-linking is expected to create a full interpenetrating polymer gel network (IPN) of gelatin and alginate, where each of gelatin and alginate is separately in-situ cross-linked by mTG and calcium, respectively.

The alginate is preferably a sodium salt of alginic acid, having enough guluronate residues or G-blocks to support ionic cross-linking by calcium ions. The sodium alginate is preferably a low viscosity grade to facilitate mixing with gelatin in solution.

As an alternative to sodium alginate, low methoxyl pectin, which is known to gel in the presence of calcium ions, can be used.

The source of the calcium ions is supplied as a calcium salt. Optionally, the calcium salt can be calcium chloride, calcium gluconate, calcium sulfate or calcium carbonate. Calcium chloride crosslinks alginate more readily than other calcium salts, potentially causing it to precipitate out of solution during the preparation of the formulation. This can be circumvented by using the slow dissolving calcium gluconate or the poorly soluble calcium sulfate or calcium carbonate. The latter may act as a slow release depot for calcium ions in vivo.

Optionally the calcium salt is part of the composition. When exposed to moisture, the calcium salt is dissolved and the dissolved calcium can then induce crosslinking of the alginate within the composition. The calcium salt can combined in the foamed adhesive layer together with the gelatin, alginate and mTG. Alternatively, the calcium salt can be spatially separated from the alginate by being added to a separate part of the device, e.g. the backing layer or the bonding layer, where it serves as a depot. After application of the device in vivo and exposure to moisture, the calcium salt will dissolve and diffuse away from its original location, and when it reaches the alginate containing layer it will crosslink the alginate.

Optionally, the calcium salt may be added in situ by applying a calcium salt solution on the tissue, or on the adhesive layer of the coated mesh facing the tissue, or on the backing side facing away from the tissue, prior to adhering the coated mesh onto the tissue. Applying the calcium salt solution can be done by wetting, spreading or spraying. Alernatively, a powdered calcium salt can be applied directly as described above for the calcium solution.

According to some embodiments of the present invention, there is provided a composition comprising gelatin, transglutaminase and chitosan. Chitosan is a deacetylated chitin, which has pendant primary amine groups. Without wishing to be limited by a single hypothesis, these pendant amine groups may serve as substrates to transglutaminases and may therefore be crosslinked to the gelatin matrix.

Other optional components that may be suitable for the composition because of their ability to support the matrix formation, for example due to high molecular weight, include alginate ester, gum arabic, high viscosity carboxymethyl cellulose (CMC), xanthan gum, guar gum, pectin and PVP (polyvinylpyrrolidone), hyaluronic acid or sodium hyaluronate, alginate or pectin (without calcium). These polymers may act to increase the cohesive strength of the crosslinked matrix by virtue of their entanglement with each other or with gelatin. This phenomenon is called semi interpenetrating polymer gel network (semi-IPN), where only the gelatin is crosslinked and the additional said polymer is non-crosslinked to itself or to gelatin but is rather dispersed homogeneously throughout the pores of the crosslinked gelatin network.

According to some embodiments of the present invention, there is provided a composition comprising gelatin, transglutaminase and a PEG (polyethylene glycol) derivative capable of covalently binding to said gelatin. Optionally said PEG derivative comprises any aminated PEG derivative. Preferably, said aminated PEG derivative comprises PEG amine.

According to some embodiments of the present invention, there is provided a composition comprising gelatin, transglutaminase and a PVA (polyvinyl alcohol) derivative capable of covalently binding to said gelatin. Optionally, said PVA derivative comprises any aminated PVA derivative. Preferably, said aminated PVA derivative comprises PVA amine.

According to some embodiments of the present invention, there is provided a cross-linked composition, comprising a foamed gelatin and transglutaminase. Optionally, said transglutaminase is present in a lyophilized form.

According to some demonstrative embodiments, the methods and/or devices described herein may include in-situ cross-linking between gelatin chains and endogenous collagen of tissue ECM (extra cellular matrix), for example, to create a strong, hemostatic barrier for fluids.

In some demonstrative embodiments, the methods and/or devices described herein may include effectively affecting hemostasis and/or fluid-stasis, for example, by having Gelatin and TG applied in a lyophilized form, e.g., wherein the Gelatin and TG may be reconstituted by the blood or other body fluid. As used herein, the term "lyophilization" may optionally relate to any type of drying, including but not limited to vacuum drying. Optionally and preferably, drying is performed at a temperature that is lower than the sol-gel transition temperature (the physical gelation point) of the composition's protein matrix.

In some demonstrative embodiments, the methods and/or devices described herein may include a gelatin-TG mixture in lyophilized form, characterized, for example, by having an increased shelf life.

In some demonstrative embodiments, the methods and/or devices described herein may include gelatin and TG in layered, lyophilized form, for example, to provide more rapid reconstitution, which, in accordance with some embodiments, may be helpful for a high pressure fluid flow environment.

In some demonstrative embodiments, the methods and/or devices described herein may include a dry composition based on gelatin cross-linking technology that may mimic the natural blood-clotting cascade and/or can be used to effect hemostasis, closing and/or sealing wounds and/or incisions, reinforce staple and/or suture lines, buttress natural tissue, and/or for any other suitable medical and/or surgical applications.

In some demonstrative embodiments, the composition may comprise a gelatin or collagen matrix with an enzymatic cross-linker, preferably microbial transglutaminase, e.g., integrated into the matrix.

In some demonstrative embodiments, the methods and/or devices described herein may include dry gelatin-enzyme composition, for example, wherein the composition may form a patch.

In some demonstrative embodiments, the methods and/or devices described herein may provide a device that includes a mechanical backing layer with a gelatin-TG mixture, for example, to increase the hemostatic and/or fluid control capacity of the mixture, e.g., by slowing the fluid and/or allowing the gelatin-TG more time to cross-link and/or block the fluid leakage.

In some demonstrative embodiments, the methods and/or devices described herein may include dry gelatin-enzyme composition that may include a degradable and/or nondegradable device incorporated into the gelatin matrix, for example, such that when the composition comes into contact with fluid, the device may be adhered to a tissue surface.

In some demonstrative embodiments, the methods and/or devices described herein may include dry gelatin-enzyme composition that may include a degradable and/or nondegradable device where the device may be a surgical mesh, for example, for the reinforcement of damaged tissue.

In another embodiment, non-cross-linked gelatin or mTG may be present together with partially cross-linked gelatin-mTG.

In another embodiment, non-cross-linked gelatin or mTG may be present together with cross-linked gelatin-mTG.

In another embodiment, a non-cross-linked gelatin is present together with a mTG.

While a number of surgical adhesives are currently used in the surgical arena, no existing commercially available product is provided in a suitable device that features a fixatable hernia mesh. Furthermore, no existing commercially available product can provide sufficient adhesive strength, as part of an implantable medical device, to ensure adherence to tissue sites while allowing for rapid tissue ingrowth.

There is provided a method of treating a target tissue (not defined by the scope of the claims), comprising applying to the tissue a composition comprising collagen or a collagen derivative and a non-toxic cross-linking agent, in the form of a patch, optionally comprising a mesh, optionally according to any embodiment herein.

Optionally, the non-toxic cross-linking agent may include one or more enzymes and/or an enzymatic composition. The one or more enzymes may include transglutaminase or a transglutaminase composition. The weight ratio of gelatin to transglutaminase may be in a range of from about 50:1 to about 5000: 1. The transglutaminase composition may have a specific activity level (enzyme units/protein content) of about at least 1 U/mg. The transglutaminase may have a specific activity level of at least about 5 U/mg.

Optionally, the activity level of the transglutaminase in the gelatin-transglutaminase composition is from about 1 to about 500 U/g of gelatin. MThe activity level may be from about 5 to about 120 U/g of gelatin.

Optionally, the transglutaminase composition may comprise a plant, recombinant animal, and/or microbe derived transglutaminase other than blood derived Factor XIII. Optionally, the collagen and/or collagen-derivative may be produced from animal origin, recombinant origin or a combination thereof. The animal origin may be selected from the group consisting of fish and mammals. The mammal is selected from the group consisting of pigs and cows.

Optionally, the collagen-derivative is a gelatin.

Optionally, the gelatin is of type A (Acid Treated) or of type B (Alkaline Treated). More preferably, the gelatin comprises high molecular weight gelatin. Optionally, the gelatin has a bloom of at least about 250.

Optionally, recombinant gelatin is produced using bacterial, yeast, animal, insect, or plant systems or any type of cell culture.

Optionally, gelatin is purified to remove salts.

Optionally, wounded tissue is selected from the group consisting of surgically cut tissue, surgically repaired tissue, and traumatized tissue.

Optionally, the method may further comprise reducing bleeding and/or leakage of other bodily fluids from the tissue. Optionally a bodily fluid is selected from the group consisting of cerebral spinal fluid, intestinal fluid, air, bile, and urine. Preferably, the method further comprises inducing hemostasis or stasis of other leaking bodily fluids in the tissue.

Optionally, the wound is bleeding or leaking another bodily fluid and treating the wounded tissue comprises applying the composition to the wound site, for example in the form of a patch, to encourage in situ cross-linking between gelatin chains and the endogenous collagen of tissue extra-cellular matrix to create a barrier to fluid leakage or bleeding. Preferably, the non-toxic cross-linking agent comprises transglutaminase.

Transglutaminase may optionally be extracted from one or more of Streptoverticillium mobaraense, Streptoverticillium Baldaccii, a Streptomyces Hygroscopicus strain, or Escherichia Coli.

Optionally, the transglutaminase comprises a plant, recombinant, animal, or microbe derived transglutaminase other than blood derived Factor XIII. Preferably, the composition further comprises a stabilizer or filler. Also preferably, the composition has a pH in a range of from about 5 to about 8.

Optionally, gelatin is produced from animal origin, recombinant origin or a combination thereof. Preferably, the animal origin is selected from the group consisting of fish and mammals. More preferably, the mammal is selected from the group consisting of pigs and cows. Most preferably, the gelatin comprises pig skins or pig bones, or a combination thereof. Also most preferably, the gelatin is of type A (Acid Treated) or of type B (Alkaline Treated). Also most preferably, the gelatin comprises high molecular weight gelatin.

Optionally, the gelatin has a bloom of at least about 250. Preferably, the fish comprises a cold water species of fish.

Optionally, recombinant gelatin is produced using bacterial, yeast, animal, insect, or plant systems or any type of cell culture.

Optionally, gelatin is purified to remove salts.

Optionally, gelatin has at least one adjusted, tailored or predetermined characteristic.

Optionally the composition further comprises an additional hemostatic agent. Preferably the additional hemostatic agent further comprises one or more of albumin, collagen, fibrin, thrombin, chitosan, ferric sulfate, or other metal sulfates.

According to some embodiments of the present invention, there is provided a composition comprising a cross-linkable protein or polypeptide, with the proviso that said protein or polypeptide is not fibrin, a calcium independent enzyme which induces cross-linking of said cross-linkable protein, a denaturing agent and an agent for reversing an effect of said denaturing agent, for reversing sol gel transition point lowering effect of the denaturing agent.

Optionally said denaturing agent comprises urea and said agent for reversing said effect of said denaturing agent comprises urease.

Optionally any composition as described herein may further comprise sorbitol. Optionally and preferably, said sorbitol is present in a sufficient amount to increase the cross-linked composition's flexibility and/or to accelerate the rate of cross-linking. The composition may also optionally further comprise acetate.

According to some embodiments of the present invention, any of the compositions herein may optionally further comprise a plasticizer. Optionally, said plasticizer is selected from the group consisting of Gum Arabic, Guar Gum, PVA, Polyvinylpyrrolidone (PVP), citric acid alkyl esters, glycerol esters, phthalic acid alkyl esters, sebacic acid alkyl esters, sucrose esters, sorbitan esters, acetylated monoglycerides, glycerols, fatty acid esters, glycols, propylene glycol, lauric acid, sucrose, glyceryl triacetate, poloxamers, diethyl phthalate, mono- and di-glycerides of edible fats or oils, dibutyl phthalate, dibutyl sebacate, polysorbate, polyethylene glycols 200 to 12,000, Carbowax polyethylene glycols.

According to some embodiments of the present invention, any of the compositions herein may optionally further comprise a surfactant. Said surfactant comprises a polyoxyethylene-sorbitan-fatty acid ester, polyoxyethyleneglycol dodecyl ether, polyoxyethylene-polyoxypropylene block copolymer, sodium lauryl sulfate, sodium dodecyl sulfate, sodium laureth sulfate, sodium lauryl ether sulfate, poloxamers, poloxamines, alkyl polyglucosides, fatty alcohols, fatty acid salts, cocamide monoethanolamine, and cocamide diethanolamine.

More preferably, a concentration of said surfactant is in the range of from about 0.1% to about 5% w/w of dry weight of said cross-linkable protein. Optionally and most preferably, said polyoxyethylene-sorbitan-fatty acid ester comprises one or more of polysorbates 20, 21, 0, 60, 61, 65, 80 or 85.

According to some embodiments of the present invention, for any of the compositions herein, optionally said enzyme comprises transglutaminase, the composition further comprising one or more of Cystamine, Cysteine, cyanate or Melanin.

According to some embodiments of the present invention, any of the compositions herein may optionally further comprise an ammonia scavenging, sequestering or binding agent, a stimulator of ammonia metabolism, or an inhibitor of cellular ammonia uptake. Optionally, said ammonia scavenging agent comprises disaccharide lactulose. Also optionally, said ammonia-binding agent comprises a saponin. Preferably, said ammonia scavenger comprises a solution comprising sodium phenylacetate and sodium benzoate.

Also preferably, said stimulator of ammonia metabolism comprises L-glutamine, L-glutamate, or a combination thereof.

Also preferably, said inhibitor of cellular ammonia uptake comprises L-glutamine, L-glutamate, or a combination thereof.

According to some embodiments of the present invention, there is provided a microbial transglutaminase ("mTG") composition with specific activity >25 enzyme units per milligram, >95% electrophoretic purity, <5 endotoxin units per gram, and <10 CFU/g. Such a transglutaminase may optionally be provided as the cross-linker of any of the above claims.

Optionally the mesh based composition comprises a reinforcing backing layer and a surgical mesh, wherein said surgical mesh is located between the reinforcement layer and the gelatin matrix; in the middle of the gelatin matrix; or on top of the gelatin matrix; or a combination thereof.

Optionally the mesh based composition comprises a mesh without the backing layer.

Optionally the cross-linkable protein includes a plurality of moieties, and wherein more than 50% of said moieties are non-cross linked. Optionally the product of further comprises a reinforcing back layer, wherein said reinforcing back layer comprises a resorbable material. Optionally said resorbable material is selected from the group consisting of cellulose (e.g. Hydroxy propyl methyl cellulose = "HPMC"), oxidized cellulose, proteinaceous substance, such as fibrin, keratin, collagen and/or gelatin, or a carbohydrate substances, such as alginates, chitin, cellulose, proteoglycans (e.g. poly-N-acetyl glucosamine), glycolic acid polymers, lactic acid polymers, or glycolic acid/lactic acid copolymers.

Optionally, during fabrication of the patch, the gelatin/mTG solution has a lower than neutral pH. The inhibition of the mTG enzyme throughout the "wet" part of the mesh preparation process through reduction in pH value helps prevent premature cross-linking of the gelatin solution. The pH value may optionally comprise a value within a range of pH values of 3 to 5, preferably 3.3 to 4.3, more preferably 3.6 to 4.0.

A porous adhesion layer may be created with pressurized gas, in which gas is mixed with the solution containing the cross-linking substrate and the cross-linking material, such as gelatin and mTG for example. The foamed solution may then optionally be extruded onto a substrate, such as PEEK (poly ether ether ketone) for example .

Another method of creating a porous sponge-like structure featuring a dry mixture of gelatin-enzyme is using the freeze-drying method (optionally without additional means of aeration). The process preferably involves rapid cooling to near zero, zero or sub-zero temperatures (Centigrade) of a solution containing the cross-linking substrate and the cross-linking material, such as gelatin and mTG for example. Pressure on the solution is then reduced so that the liquid of the solution sublimates, leaving a freeze dried material .

Specific surgical mesh positioning in regard to the gelatin foam, according to at least some embodiments, may increase desirable properties, including with regard to tissue intergration. Surprisingly, the inventors have found that the position of the mesh within the foam coating (that is, completely surrounded by foam) may reduce the degree of tissue response to the prosthesis at initial stages following product application on the tissue. Preferably, the mesh is positioned so that only part of it is coated by foam while other part is not coated by foam, allowing fast tissue response to the surgical mesh.

European Patent EP2219691 describes a bandage in which the HPMC layer is designed to prevent adhesions to visceral organs, not as a separate layer that protects the product or prevents the adhesive layer from sticking to gloves, viscera etc.

United States Patent Application 20140271781 describes a tooth whitening adhesive gel with a non adhesive backing comprised of cellulose esters; however such a compositional structure is clearly irrelevant for wound treatment.

US Patent 8703177 describes a mucoadhesive patch comprised of an adhesive layer and a backing layer which is non-adhesive and which can further include at least one water erodable, film-forming polymer (such as HPMC). Again such a patch would clearly be irrelevant for surgical treatment.

As used herein, "about" means plus or minus approximately ten percent of the indicated value.

Other features and advantages of the various embodiments of the invention will be apparent from the following detailed description, and from the claims.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. The present invention is defined by the claims.

In the drawings:
Fig. 1 shows a top view of the device design, including a section of adhesive covered mesh and a section of only adhesive surrounding the mesh-adhesive section.
Fig. 2A shows a side view of the device portraying an optional placement of the mesh within the adhesive, in a way that the adhesive surrounds the mesh in all directions, while Figure 2B shows an exploded view of the layers.
Fig. 3 shows the measurement of relative tackiness of the various backing and composition combinations with an Instron.
Fig. 4 shows an exemplary device implanted in vivo. An embedded surgical hernia mesh is strongly fixated to a peritoneal tissue.
Fig. 5 shows an optional and non-limiting method of preparation of the composition.
Fig. 6 shows some optional dimensions, minimum and maximum, for various mesh based composition shapes. See Figure 2A for a description on how the dimensions given relate to the product.
Figure 7 shows the effect of pH on mTG activity by viscometer test. 9% gelatin in pH 5.5 (native), 4 and 3.5 were mixed with 40u/ml enzyme solution at 37°c (2:1) Figure 8 shows a schematic view of the aeration and mixing system;
Figure 9 shows an SEM image of a Cross-section of a gelatin-mTG dry foam article, prepared by the process described in example 7;
Figure 10 shows an SEM image of the adhesive surface of a gelatin-mTG dry foam article, prepared by the process described in example 7.
Figures 11A and 11B show SEM images of articles prepared as described herein: Figure 11A shows an SEM image of a cross-section of LM-147 article - Mesh in embedded in the middle of the foam, while Figure 11B shows an SEM image of a cross-section of LM-149 article - Surgical mesh is located at the bottom (surface);
Figures 12A and 12B show SEM images of the bottom of the articles of Figure 11: Figure 12A shows an SEM image of the bottom surface of LM-147 article - Surgical mesh is not visible as it is fully covered by foam, while Figure 12B shows an SEM image of the bottom surface of LM-149 article - Surgical mesh is located at the surface level, not fully covered;
Figures 13A and 13B show SEM images of the articles of Figure 11, fixated on collagen: Figure 13A shows an SEM image of LM-149 after fixation on collagen as a tissue simulating substrate (collagen is the bottom layer), while Figure 13B shows an SEM image of LM-147 after fixation on collagen as a tissue simulating substrate (collagen is the top layer);
Figure 14 shows the fixation strength of the different models on collagen, as tested by the lap shear method, 4 minutes after fixation;
Figure 15 shows the fixation strength of the different models on swine peritoneum tissue, as tested by the lap shear method;
Figure 16 shows representative histopathology images of samples explanted after 7 days from swine;
Figure 17 shows a boxplot representing % surface area of Control mesh (bare Surgical Mesh) and Mesh based compositions (Surgical mesh covered with adhesive) groups GF-199 (Crosslinked gelatin), GF-200 (HPMC) covered by adhesions, 14 days after implantation;
Figure 18 shows the fixation strength of Gelatin - Alginate mesh based composition (batch No. GF-502) on collagen, as tested by the lap shear method, 24 hours after fixation. GF-502 articles were immersed for 24 hours post application on collagen in either 0.9% saline or 0.9% saline with addition of 50mM CaCl₂;
Figure 19 shows the fixation strength of Gelatin- Chitosan mesh based compositions (batches No. GF-510, GF-511, GF-512) on collagen, as tested by the lap shear method, 24 hours after fixation. The articles were immersed for 24 hours post application on collagen in 0.9% saline.

### DESCRIPTION OF THE PREFERRED EMBODIMENTS

The present invention is of mesh-based compositions comprising a solution of a cross-linkable protein or polypeptide, and an agent which induces cross-linking of the cross-linkable protein.

The present invention provides a mesh-based composition comprising a cross-linkable protein or polypeptide and one or more cross-linking materials according to claim 1.

The mesh comprises a self-adhering surgical mesh which requires no additional fixation means while at the same time minimizes adhesions. The mesh comprises a composite mesh featuring a foamed composition of the cross-linkable protein and a non-toxic material that induces cross-linking of the cross-linkable material as disclosed in claim 1. The composite mesh may optionally be in a sheet-like form.

According to at least some embodiments, preferably the composite mesh features an incorporated hernia mesh. The structure features two sections, one of which is composed of a mesh enclosed within the adhesive composition and another which contains the adhesive composition alone, without mesh within it. This design allows for a self-fixating adhesion minimizing hernia mesh device, preventing the mesh from shifting, migrating, rolling up its edges, or changing its position without the use of sutures, staples and other additional means of fixation.

Optionally and preferably, the cross-linkable protein includes gelatin and any gelatin variant or variant protein as described herein. Optionally and preferably, the non-toxic material comprises transglutaminase (TG), which may optionally comprise any type of calcium dependent or independent transglutaminase, which may for example optionally be a calcium-independent microbial transglutaminase (mTG). Without wishing to be limited in any way, among the improved properties of at least some embodiments of the present invention, the compositions of the present invention provide an increased rate of protein cross-linking as compared to background art compositions. Furthermore, the crosslinking reaction of mTG represents a significant improvement over that catalyzed by Factor XIIIa of the blood coagulation system. Unlike Factor XIIIa, the microbial enzyme requires neither thrombin nor calcium for activity.

Various embodiments of the present invention are described in greater detail below, under section headings which are provided for the sake of clarity only and without any intention of being limiting in any way.

### Gelatin and Transglutaminase

According to preferred embodiments of the present invention, there is provided a mesh-based composition in which the cross-linking material comprises transglutaminase and the cross-linkable protein comprises gelatin.

According to a preferred embodiment, transglutaminase is present at a specific activity level of at least about 5 U/mg.

Suitable gelatin and transglutaminase can be obtained by any of the methods known and available to those skilled in the art. Gelatin may optionally comprise any type of gelatin which comprises protein that is known in the art, preferably including but not limited to gelatin obtained by partial hydrolysis of animal tissue and/or collagen obtained from animal tissue, including but not limited to animal skin, connective tissue (including but not limited to ligaments, cartilage and the like), antlers or horns and the like, and/or bones, and/or fish scales and/or bones or other components; and/or a recombinant gelatin produced using bacterial, yeast, animal, insect, or plant systems or any type of cell culture.

According to preferred embodiments of the present invention, gelatin from animal origins preferably comprises gelatin from mammalian origins and more preferably comprises one or more of pork skins, pork and cattle bones, or split cattle hides, or any other pig or bovine source. More preferably, such gelatin comprises porcine gelatin since it has a lower rate of anaphylaxis. Gelatin from animal origins may optionally be of type A (Acid Treated) or of type B (Alkaline Treated), though it is preferably type A.

Preferably, gelatin from animal origins comprises gelatin obtained during the first extraction, which is generally performed at lower temperatures (50-60° C., although this exact temperature range is not necessarily a limitation). Gelatin produced in this manner will be in the range of 250-300 bloom and has a high molecular weight of at least about 95-100 kDa. Preferably, 275-300 bloom gelatin is used.

A non-limiting example of a producer of such gelatins is PB Gelatins (Tessenderlo Group, Belgium).

According to some embodiments of the present invention, gelatin from animal origins optionally comprises gelatin from fish. Optionally any type of fish may be used, preferably a cold water variety of fish such as carp, cod, or pike, or tuna. The pH of this gelatin (measured in a 10% solution) preferably ranges from 4-6.

Cold water fish gelatin forms a solution in water at 10° C. and thus all cold water fish gelatin are considered to be 0 bloom. For the current invention, a high molecular weight cold water fish gelatin is preferably used, more preferably including a molecular weight of at least about 95-100 kDa. This is equivalent to the molecular weight of a 250-300 bloom animal gelatin. A non-limiting example of a producer of such a gelatin is Norland Products (Cranbury, N.J.).

In a preferred embodiment of the invention, the gelatin is purified to remove salts. This can be accomplished according to previously described techniques. One such technique involves forming a 20% w/v solution of gelatin in water and heating it to 60° C. under stirring. The mixture is then let to stand still overnight. The gel obtained is dialysed against repeated changes of deionized water to eliminate salts, stirred and heated to 50° C. to disaggregate the physical network. The final solution was filtered and freeze-dried. (Crescenzi V, Francescangeli A, Taglienti A. (2002). Biomacromolecules. 3:1384-1391). Alternatively, the gelatin can be desalted by size exclusion column.

According to some embodiments of the present invention, a recombinant gelatin is used. Recombinant gelatins are currently commercially produced by FibroGen (San Francisco, Calif.). The currently preferred method is using a recombinant yeast system (Pichia Pastoris) to express specified fragments of Type I, alpha1 human sequence collagen.

In an optional but preferred embodiment of the present invention, recombinant gelatins are fully synthetic molecules, containing no contaminating components from humans or any animals. By "synthetic" it is meant that the gelatin is preferably produced according to a method selected from chemical synthesis, cell free protein synthesis, cell tissue culture, any type of bacterial, insect or yeast culture, or in plants. The use of synthetic gelatins eliminates many of the variables and drawbacks associated with tissue-derived materials, including provoking unwanted immune responses. For example, fish gelatins demonstrate high allergenicity and animal gelatins demonstrate low-moderate allergencity, while recombinant gelatins can have zero allergenicity. In human safety studies, no adverse events related to recombinant gelatin were found.

Methods of creating recombinant gelatins and the benefits of their use are fully described in U.S. Pat. Nos. 6,413,742 and 6,992,172.Recombinant gelatins can be produced to be highly (99%) purified. Recombinant gelatin production allows for the optional production of gelatins with at least one defined and predetermined characteristic, including but not limited to defined molecular weights, pI (isoelectric point), guaranteed lot-to-lot reproducibility, and the ability to tailor the molecule to match a specific application.

An example of tailoring a molecule to match a specific application has been previously described wherein a gelatin was created to be highly hydrophilic (Werten M W T, et al. (2001). Protein Engineering. 14 (6): 447-454). Optionally and preferably a gelatin according to the present invention comprises a gelatin having at least one adjusted, tailored or predetermined characteristic.

The gelatin employed in the device can be a gelatin complex or any gelatin, or a derivative or metabolite thereof, or a gelatin produced according to a single process or a plurality of processes. For example, the gelatin may optionally comprise gelatin type A or gelatin type B, or a combination thereof.

The transglutaminase may optionally comprise any plant, animal, or microbe derived transglutaminase, preferably other than blood derived Factor XIII. Preferably, microbial transglutaminase (mTG) derived from Streptoverticillium mobaraensis is used.

The transglutaminase may optionally be in a composition comprising at least one other substance, such as a stabilizer or filler for example. Non-limiting examples of such materials include maltodextrin, hydrolyzed skim milk protein or any other protein substance, sodium chloride, safflower oil, trisodium phosphate, sodium caseinate or lactose, or a combination thereof.

Transglutaminase features a negative temperature coefficient. Over the temperature range of the transglutaminase activity, it takes a shorter time to react at higher temperatures and longer amount of time to start functioning at lower temperatures. The following table 1 shows different reaction times at different temperatures comparing the same reaction grade as the reaction at 50° C., pH 6.0 that occurs in 10 minutes:

**Table 1 showing reaction temperatures of transglutaminase.**

| | | | | | |
|---|---|---|---|---|---|
| Temperature | | | | | |
| | 5° C. | 15° C. | 20° C. | 30° C. | 40° C. |
| Time (minutes) | 240 | 105 | 70 | 35 | 20 |

Non-limiting examples of commercially available transglutaminase products include those produced by Ajinomoto Co. (Kawasaki, Japan). A preferred example of such a product from this company is the Activa TG-TI (In Europe: Activa WM)-Ingredients: mTG and maltodextrin; Activity: 81-135 U/g of Activa. Other non-limiting examples of suitable products from this company include Activa TG-FP (ingredients: hydrolyzed skim milk protein, mTG; activity: 34-65 U/g of Activa TG-FP); Activa TG-GS (ingredients: sodium chloride, gelatin, trisodium phosphate, maltodextrin, mTG, and safflower oil (processing aid); activity: 47-82 U/g of Activa TG-GS); Active TG-RM (In Europe: Activa EB)-ingredients: sodium caseinate, maltodextrin, and mTG; activity: 34-65 U/g of Activa; Activa MP (ingredients: mTG, Lactose and Maltodextrin; activity: 78-126 U/g of Activa).

Other non-limiting examples of commercially available transglutaminase products include those produced by Yiming Biological Products Co. (Jiangsu, China). A preferred example of such a product from this company is the TG-B (ingredients: 1% mTG, 99% co-protein; activity: 80-130 U/g of TG-B). Other non-limiting examples of suitable products from this company include TG-A (ingredients: 0.5% mTG, 99.5% co-protein; activity: 40-65 U/g of TG-A).

For both examples, preferred transglutaminase products are those with the highest specific activity and simplest co-ingredients, as they are believed (without wishing to be limited by a single hypothesis) to have the best reactivity upon application and a lower potential for undesired side effects.

In another embodiment, a transglutaminase may optionally be extracted from Streptoverticillium Baldaccii or a Streptomyces Hygroscopicus strain to produce enzyme variants that have been shown to function optimally at lower temperatures (approximately 37° C. and 37°-45° C., respectively) (Negus SS. A Novel Microbial Transglutaminase Derived from Streptoverticillium Baldaccii. PhD Thesis. School of Biomolecular and Biomedical Science. Griffith University, Queensland, Australia and Cui L et al. Purification and characterization of transglutaminase from a newly isolated Streptomyces hygroscopicus. 2007: 105(2). p. 612-618.). Higher specific activity at lower temperatures is desirable for achieving faster and stronger cross linking of the gelatin under ambient conditions.

According to some embodiments, transglutaminase can be used in the form of any of the above described compositions, optionally including any of the commercially available mixtures that include transglutaminase.

In another embodiment, any of the above transglutaminase mixtures may optionally be purified by means of gel filtration, cation-exchange chromatography, hollow fiber filtration, or tangential flow filtration to remove their carrier proteins and/or carbohydrates. Some of these methods have been previously described (Bertoni F, Barbani N, Giusti P, Ciardelli G. Transglutaminase reactivity with gelatine: perspective applications in tissue engineering. Biotechnol Lett (2006) 28:697-702) (Broderick E P, et al. Enzymatic Stabilization of Gelatin-Based Scaffolds J Biomed Mater Res 72B: 37-42, 2005). The filter pore size used for filtration is preferably approximately 10 kDA.

Preferably, the transglutaminase is purified in a process that includes cation-exchange chromatography, hydrophobic chromatography, and ultrafiltration, as described more fully in for example US Patent No. 8367388, filed on Jun. 18, 2009, owned in common with the present application and having at least some inventors in common with the present application.

Regardless, the activity of transglutaminase is preferably measured prior to use and/or manufacture of a composition according to the present invention with a transglutaminase reactivity assay. Such an assay may optionally include but is not limited to the Hydroxamate Method, Nessler's Assay, a Colorimetric Assay, or any other assay of transglutaminase activity (see for example Folk J E, Cole P W. Transglutaminase: mechanistic features of the active site as determined by kinetic and inhibitor studies. Biochim Biophys Acta. 1966; 122:244-64; or the Nessler Assay as described in: Bertoni F, Barbani N, Giusti P, Ciardelli G. Transglutaminase reactivity with gelatine: perspective applications in tissue engineering. Biotechnol Lett (2006) 28:697-702).

In general, the purity and/or quality of the gelatin and/or the transglutaminase for use in the device (tissue adhesive, hemostatic or sealing product) composition will be of an appropriate purity known to one of ordinary skill in the relevant art to lead to efficacy and stability of the protein.

### Enzyme Purification and Concentration

According to some embodiments of the present invention, transglutaminase solutions undergo one-stage or multiple-stage purification to perform one or more of 1) removing fermentation residue from the transglutaminase mixture; 2) concentrating the amount of active translglutaminase in a transglutaminase solution; 3) further purifying the transglutaminse solution from carrier proteins or carbohydrates; 4) lowering the endotoxin level of the transglutaminase solution; and/or 5) removing all microbes from the transglutaminase solution, effectively sterilizing the solution; all without wishing to be limited to a closed list.

According to some embodiments, the solution of cross-linking material is filtered prior to mixing with the cross-linkable protein of polypeptide.

In an embodiment of the present invention, the filtration process first uses coarse filtration, sometimes known as clarification, to remove large blocks of fermentation residue. Non-limiting examples of such coarse filtration features a pore size above 0.22 µm, such as for example from about 0.45µm pore size filtration, optionally including about 0.65µm pore size filtration.

According to another embodiment of the present invention, the solution of cross-linking material is optionally and preferably passed through a filter of pore size of below 0.22 µm in a secondary filtration process after coarse filtration, for example to reduce the bioburden of the material below 10 colony forming units (CFU) per gram and make it appropriate for medical use. Preferably, the bioburden is practically eliminated to achieve a sterility assurance level (SAL) of less than about10⁻² and more preferably less than about 10⁻³, where SAL is a term used in microbiology to describe the probability of a single unit being non-sterile after it has been subjected to a sterilization process.

According to another embodiment of the present invention, either tangential flow or hollow fiber ultra-filtration techniques are used after such a secondary filtration stage, not only to purify the solution of cross-linking material by removal of carrier carbohydrates and proteins, but also to concentrate the solution. Preferred pore sizes for use with this invention are those with pore sizes larger than the size of the components of the cross-linking composition.

In an embodiment, the crosslinking material is mTG and the pore size is in the range of 10-50 kDa. In a more preferred embodiment, the crosslinking material is mTG and the pore sizes are in the range of 10-30 kDa.

According to another embodiment, one or more size exclusion chromatography steps is used to selectively separate the crosslinking material from surrounding substances.

According to another embodiment, one or more hydrophobic or hydrophilic interaction chromatography steps is used to selectively separate the crosslinking material from surrounding substances.

According to another embodiment of the present invention, the crosslinking material is a protein and one or more ion exchange chromatography steps is used to preferentially bind the crosslinking protein, thereby purifying it from the surrounding materials.

According to a more preferred embodiment, the crosslinking protein is mTG and one or cation exchange chromatography steps is used to purify the mTG.

In a preferred embodiment, the cation exchange resin is a sepharose resin.

According to another preferred embodiment, purification reduces the endotoxin level of the crosslinking material to <5 endotoxin units (EU) per gram.

According to another preferred embodiment, the crosslinking material is mTG and purification results in an mTG composition wherein the specific activity is greater than 20 enzyme units per milligram and preferably greater than 25 units per milligram.

According to another preferred embodiment, the crosslinking material is mTG and purification results in electrophoretic purity of at least 95% and preferably of at least 98%.

An mTG purification process, as a non-limiting example, is described herein that purifies a food-grade mTG product to produce an mTG composition with specific activity >25 enzyme units per milligram, >95% electrophoretic purity, <5 endotoxin units per gram, and <10 CFU/g.

As described above, mTG concentration is also a preferred parameter for some embodiments of the composition of the present invention. The above purification processes may also result in more concentrated mTG material. In addition to cross-linking gelatin more rapidly than non-concentrated mTG solutions, concentrated mTG solutions formed gels that were more elastic, more adhesive, and more transparent compared to the non-concentrated controls.

One or more supplements can also be contained in the tissue adhesive, hemostatic or sealing product, e.g., drugs such as growth factors, polyclonal and monoclonal antibodies and other compounds. Illustrative examples of such supplements include, but are not limited to: antibiotics, such as tetracycline and ciprofloxacin, amoxicillin, and metronidazole; anticoagulants, such as activated protein C, heparin, prostracyclin (PGI2), prostaglandins, leukotrienes, antitransglutaminase III, ADPase, and plasminogen activator; steroids, such as dexamethasone, inhibitors of prostacyclin, prostaglandins, leukotrienes and/or kinins to inhibit inflammation; cardiovascular drugs, such as calcium channel blockers, vasodilators and vasoconstrictors; chemoattractants; local anesthetics such as bupivacaine; and antiproliferative/antitumor drugs such as 5-fluorouracil (5-FU), taxol and/or taxotere; antivirals, such as gangcyclovir, zidovudine, amantidine, vidarabine, ribaravin, trifluridine, acyclovir, dideoxyuridine and antibodies to viral components or gene products; cytokines, such as alpha- or beta- or gamma-Interferon, alpha- or beta-tumor necrosis factor, and interleukins; colony stimulating factors; erythropoietin; antifungals, such as diflucan, ketaconizole and nystatin; antiparasitic agents, such as pentamidine; anti-inflammatory agents, such as alpha-1-anti-trypsin and alpha-1-antichymotrypsin; anesthetics, such as bupivacaine; analgesics; antiseptics; and hormones. Other illustrative supplements include, but are not limited to: vitamins and other nutritional supplements; glycoproteins; fibronectin; peptides and proteins; carbohydrates (both simple and/or complex); proteoglycans; antiangiogenins; antigens; lipids or liposomes; and oligonucleotides (sense and/or antisense DNA and/or RNA).

### Illustrative compositions

The above described cross-linking substrates and cross-linking materials may optionally be combined with one or more additional materials to form various compositions according to the present invention, for use with a patch as described herein. According to some embodiments, the adhesive material optionally and preferably comprises: (i) gelatin; (ii) a transglutaminase. More preferably, the gelatin and transglutaminase are provided in sufficient quantities to be useful as a tissue adhesive, sealing, or hemostatic agent.

In addition, one or more supplements can also be contained in the tissue adhesive, sealing, or hemostatic product, e.g., drugs such as growth factors, polyclonal and monoclonal antibodies and other compounds. Illustrative examples of such supplements include, but are not limited to: antibiotics, such as tetracycline and ciprofloxacin, amoxicillin, and metronidazole; anticoagulants, such as activated protein C, heparin, prostracyclin (PGI₂), prostaglandins, leukotrienes, antitransglutaminase III, ADPase, and plasminogen activator; steroids, such as dexamethasone, inhibitors of prostacyclin, prostaglandins, leukotrienes and/or kinins to inhibit inflammation; cardiovascular drugs, such as calcium channel blockers, vasodilators and vasoconstrictors; chemoattractants; local anesthetics such as bupivacaine; and antiproliferative/antitumor drugs such as 5-fluorouracil (5-FU), taxol and/or taxotere; antivirals, such as gangcyclovir, zidovudine, amantidine, vidarabine, ribaravin, trifluridine, acyclovir, dideoxyuridine and antibodies to viral components or gene products; cytokines, such as alpha- or beta- or gamma-Interferon, alpha- or beta-tumor necrosis factor, and interleukins; colony stimulating factors; erythropoietin; antifungals, such as diflucan, ketaconizole and nystatin; antiparasitic agents, such as pentamidine; anti-inflammatory agents, such as alpha-1-anti-trypsin and alpha-1-antichymotrypsin; anesthetics, such as bupivacaine; analgesics; antiseptics; and hormones. Other illustrative supplements include, but are not limited to: vitamins and other nutritional supplements; glycoproteins; fibronectin; peptides and proteins; carbohydrates (both simple and/or complex); proteoglycans; antiangiogenins; antigens; lipids or liposomes; and oligonucleotides (sense and/or antisense DNA and/or RNA).

### Mesh-based Composition and Structure

According to at least some embodiments, the present invention is of a temporary resorbable anti adhesive non-stick backing. It is designed to protect medical devices from sticking to gloves, surgical tools and internal organs.

Examples are pads, foams, bandages used for hemostasis or sealing purposes. Other examples are coated surgical meshes. Some of these devices contain or are coated with an adhesive layer that fixes the device by adhering it to a tissue. If the same adhesive is present on the side of the device that is facing away from the tissue it might become tacky and stick to gloves, surgical tools and internal organs. In addition, if the coating layers are not tacky by themselves, during the surgery they could still stick to gloves, surgical tools and internal organs after the latter become moist or wet by contact with peritoneal fluids, blood or saline.

In another scenario, during device handling, the coating might unintentionally touch a moist tissue such as the intestines and stick to it before the surgeon has the opportunity to position the device in the desired location. The non-stick backing is designed to prevent such an occurrence.

The device backing may be composed of any polymeric material, of natural, semisynthetic or synthetic nature, that is soluble in water to some extent such as polysaccharides, proteins etc.

The backing is required for the short duration in which there is a risk of the device coating sticking to unwanted surfaces or tissues, for example during handling of the device or during the surgery. Once the device has been placed and secured at the desired location the backing is no longer required, and therefore was designed to dissolve quickly. The backing can be made of cellulose ether derivatives, such as HPMC (hydroxypropyl methylcellulose) or HPC (hydroxypropyl cellulose), HEC (hydroxyethyl cellulose) or EC (ethyl cellulose). The backing can be also made from crosslinked gelatin (enzymatic, physical or chemical crosslinking).

According to at least some embodiments of the present invention, there is provided a patch, comprising a gelatin layer and a reinforcing back layer, wherein said gelatin layer comprises gelatin and an enzyme integrated into a carrier selected from a group consisting of: HPC (hydroxypropyl cellulose), HPMC (hydroxypropyl methylcellulose), carboxymethyl cellulose, hydroxylethyl cellulose, ethylcellulose, PVP (polyvinyl pyrrolidone), PVA (polyvinyl alcohol), PEG (polyethylene glycol), PEI (polyethyleneimine), starch, microcrystalline cellulose, oxidized cellulose.

The molecular weight of the polymer may optionally be selected in order to determine the performance of the backing. Without wishing to be limited by a single hypothesis, it is believed that small molecular weight results in faster dissolution of the polymer in moist/wet conditions- thus resulting in development of tackiness in the backing itself. The larger the molecular weight, the less soluble the polymer is and as a result the tendency for tackiness is reduced.

The non-adhesive backing layer may comprise a water-erodable, film-forming pharmaceutically acceptable polymer such as hydroxyethyl cellulose, hydroxypropyl cellulose, hydroxypropylmethyl cellulose, hydroxyethylmethyl cellulose, polyvinylalcohol, polyethylene glycol, polyethylene oxide, ethylene oxide-propylene oxide copolymers, collagen and derivatives, gelatin, albumin, polyaminoacids and derivatives, polyphosphazenes, polysaccharides and derivatives, chitin and chitosan, alone or in combination. The backing layer component may or may not be crosslinked depending on the desired erosion kinetics.

In one embodiment, the preferred backing layer component comprises Hydroxypropyl Methylcellulose (HPMC). Preferably, in the case of hydroxyethyl cellulose, the average molecular weight (Mw estimated from intrinsic viscosity measurements) is in the range 2x104 to 1.2x106, and more preferably in the range 2.5x105 to 1x106.

In another embodiment, it is possible to mix two HPMC polymers differ in MW in order to achieve the desired mechanical properties or film thickness. For example, using a 0.5-2% of HPMC (1×10^6 Da) mixed with 1-4% of HPMC (4×10^5 Da) is contemplated within at least some embodiments of the present invention.

The erosion kinetics of the backing layer may optionally be altered in many different ways in order to modify the residence time.

Optionally, such alteration may be performed with a combination which comprises hydroxypropyl methylcellulose and an alkylcellulose such as ethylcellulose. Such a combination comprises a film-forming amount of alkylcellulose, hydroxypropyl methylcellulose, and a suitable solvent. Advantageously, the characteristics of the film formed from the gel may be modified depending upon the ratio of hydroxypropyl methylcellulose to alkylcellulose. Such modifiable characteristics advantageously include the kinetics of erodability.

Typically, the ratio of hydroxypropyl methylcellulose to alkylcellulose is that necessary to form a suitable film. This ratio may vary based on the other components and the type of alkylcellulose. However, if ethylcellulose is employed then the ratio of hydroxypropyl cellulose to ethyl cellulose is usually from about 1000:1 to about 3:1, preferably from about 200:1 to about 4:1, more preferably from about 200: 1 to about 8:1. Typically, as the ratio of hydroxypropyl cellulose to alkylcellulose increases, the water erodability increases, i.e., the films are more readily washed away. Thus, the ethylcellulose is a component which acts to adjust the kinetics of erodability of the device.

According to another embodiment the backing layer is designed to remain in place for a longer duration, e.g. up to 1 month following implantation, where it may reduce adhesions of the mesh or the adhesive layer to the visceral organs. In this case the backing layer may only dissolve very slowly, this can be achived by crosslinking the polymer which comprise the backing.

Crosslinking the film-forming polymer may also optionally be performed to affect its properties. Crosslinking agents known in the art are appropriate for use in the invention and may include glyoxal, propylene glycol, glycerol, dihydroxy-polyethylene glycol of different sizes, butylene glycol, and combinations thereof. The amount of crosslinking agent used may vary, depending on the particular polymers and crosslinking agent but usually should not exceed 5% molar equivalent of the polymeric material, and preferably comprises 0 to 3% molar equivalent of the polymeric material.

Gelatin backing can be crosslinked by methods known to those skilled in the art, and include but are not limited chemical crosslinkers such as formaldehyde, glutaraldehude and EDC, physical methods such has DHT (dehydrothermal) treatment or enzymatic methods, e.g. by mTG of any type, including without limitation mammalian TG, or Factor XIII.

It is possible to control the mechanical properties of the backing layer by employing excipients which plasticize the film concomitantly. The excipient or plasticizer often improves the mechanical properties of the device and/or modifies the drug release profile or disintegation time. Suitable excipients or plasticizers modifying the erosion behavior of the layer(s) may include alkyl-glycol such as propylene glycol, polyethyleneglycols, oleate, sebacate, stearate or esters of glycerol, phthalate and others. Other suitable plasticizers include esters such as acetyl citrate, amyl oleate, myristyl acetate, butyl oleate and stearate, dibutyl sebacate, phthalate esters such as diethyl, dibutyl, and diethoxy ethyl phthalate and the like, fatty acids such as oleic and stearic acid, fatty alocohols such as cetyl, myristyl, and stearyl alcohol. Moreover, in some instances, a polymer, a pharmaceutical, or solvent residual may act as a plasticizer. One preferable plasticizer is PEG. The MW range is from 200 Da to 1000 Da. Prefarably, the concentration of PEG in the film is from 10% w/w to 50% w/w, more preferably from 25-40%. Another preferred plasticizer is glycerol, the concentration of glycerol in the film is from 10% w/w to 50% w/w, more preferably from 25-40% w/w.

The backing layer can be attached to the adhesive layer, by means of direct application or indirect application.

By "direct application" various methods may optionally be used, including spreading or spraying the backing in a liquid form on the gelatin layer and drying it. Using non aqueous solvents such as ethanol prevents undesired dissolution of the gelatin layer.

In yet another example of direct application the backing layer is cast as a film and dried, then the adhesive layer is applied wet on top of the said film and subjected to another round of drying, such as lyophilization.

In another embodiment, direct application may optionally be performed as follows: the backing layer can be attached to the adhesive layer by taking advantage of the inherent tackiness of polymers after they have been wetted with water, either liquid or steam. While the polymer, either the adhesive layer, backing layer, or both, are wet and/or tacky, the two layers are attached to each other and the resulting bond formed is permanent, even after the water used for activation is eliminated.

In indirect application, the backing may optionally be prepared separately as a film, e.g. by cast drying, and then attached to the adhesive layer by applying a bonding layer on the film or on the adhesive layer by spraying or spreading. The adhesive layer is then attached to the backing layer where the bonding layer glues them together. The bonding layer can be dried before or after the attachment of the gelatin layer to the backing layer.

In another embodiment, the attachment may also optionally be performed by wetting the adhesive layer or the bonding layer directly with water or other aqueous liquid such as saline or by incubating in a high humidity environment (such as humidity chamber or by steam).

The bonding layer is optionally composed of a naturally derived polysaccharide such as maltodextrin, starch, cellulosics (e.g. hydroxypropyl cellulose, hydroxypropyl methylcellulose, ethyl cellulose, carboxymethyl cellulose, sodium carboxymethyl cellulose or blended compositions thereof), or from a synthetic polymer such as PVP. The preferred MW range for PVP is from 1×10^4 Da to 1.3×10^6 Da.

A plasticizer can be added to increase the moisture content of the bonding layer and thus its tackiness. One preferable plasticizer is PEG. The MW range of PEG is from 200 Da to 1000 Da. Preferably, the concentration of PEG in the bonding layer is from 10% w/w to 50% w/w, more preferably from 25-40%. Another preferred plasticizer is glycerol, such that optionally the concentration of glycerol in the bonding layer is from 10% w/w to 50% w/w, more preferably from 25-40%. Above 50% w/w plasticizer the bonding layer becomes too wet and the backing layer might slide off the gelatin layer and below 10% w/w plasticizer it dries out too quickly.

The product features a mesh and is adapted for surgical mesh fixation where mesh can be adhered to an organ surface, tissue surface, or cavity.

Optionally said mesh comprises any degradable or non degradable material, including without limitation synthetic mesh, biological mesh, or a combination synthetic-biological mesh.

Optionally the product is adapted for inguinal, femoral, umbilical or incisional ventral hernia repair, or other types of surgical mesh reconstruction.

Optionally the product is adapted for any of large diaphragmatic hernia repair, for rectopexy (rectal prolapsed) mesh fixation, for reconstruction of a prolapsed vaginal vault, or for other pelvic floor mesh reinforcement operations (gynecology procedures).

Optionally the product further comprises an additional agent selected from the group consisting of: an antibiotic, an anticoagulant, a steroid, a cardiovascular drug, a local anesthetic, a antiproliferative/antitumor drug, an antiviral, a cytokine, colony stimulating factors; erythropoietin; an antifungal; an antiparasitic agent; anti-inflammatory agents; anesthetics, such as bupivacaine; analgesics; antiseptics; and hormones.

Optionally the product further comprises an additional agent selected from the group consisting of vitamins and other nutritional supplements; glycoproteins; fibronectin; peptides and proteins; carbohydrates (both simple and/or complex); proteoglycans; antiangiogenins; antigens; lipids or liposomes; and oligonucleotides (sense and/or antisense DNA and/or RNA).

Optionally said cytokine is selected from the group consisting of alpha- or beta- or gamma-Interferon, alpha- or beta-tumor necrosis factor, and interleukins.

Optionally said antiviral is selected from the group consisting of gangcyclovir, zidovudine, amantidine, vidarabine, ribaravin, trifluridine, acyclovir, dideoxyuridine and antibodies to viral components or gene products. Optionally said anti-tumor drug is selected from the group consisting of 5-fluorouracil (5-FU), taxol and/or taxotere.

Optionally said cardiovascular drug is selected from the group consisting of calcium channel blockers, vasodilators and vasoconstrictors; chemoattractants.

Optionally said steroid is selected from the group consisting of dexamethasone, inhibitors of prostacyclin, prostaglandins, leukotrienes and/or kinins to inhibit inflammation.

Optionally said anticoagulant is selected from the group consisting of activated protein C, heparin, prostracyclin (PGI2), prostaglandins, leukotrienes, antitransglutaminase III, ADPase, and plasminogen activator.

Optionally said antibiotic is selected from the group consisting of tetracycline, ciprofloxacin, amoxicillin, and metronidazole.

Optionally the product further comprises a wound healing agent.

Optionally the product further comprises a hemostatic agent.

There is also provided a method of producing a mesh based composition (not defined by the scope of the claims), comprising: producing a cross-linkable protein matrix, comprising a cross-linkable protein; depositing an enzymatic composition in said protein matrix, wherein said enzymatic composition comprises an enzyme capable of cross-linking said cross-linkable protein; thereby producing the product. The protein matrix may be deposited at a depth of at least 0.5 mm. The depth of deposition may be lower.

Optionally said enzyme comprises transglutaminase and said transglutaminase comprises any type of calcium dependent or independent transglutaminase. Optionally said transglutaminase comprises a microbial transglutaminase (mTG).

Optionally said cross-linkable protein comprises gelatin in the form of a gelatin solution, comprising mixing said gelatin solution in a mixer at a rate to form a foamed solution, drying said foamed solution to form a dried solution and combining said dried solution with said enzyme.

Optionally said mixing said gelatin solution comprises mixing said gelatin solution in a mixer with pressurized air, at a mixing rate and air pressure so as to foam the solution; wherein said method further comprises lyophilizing the foamed gelatin solution to form a lyophilized porous layer of gelatin.

Optionally said rate is from 100 RPM to 10,000 RPM. Optionally said rate is from 1000 RPM to 6000 RPM.

Optionally said rate is from 0.1 cm³/second to 10,000 cm³/second per volume of foam.

Optionally said cross-linkable protein comprises gelatin in the form of a gelatin solution, comprising mixing said gelatin solution with a chemical foaming agent so as to foam the solution; wherein said method further comprises drying the foamed gelatin solution to a dried porous layer of gelatin.

Optionally said chemical foaming agent comprises sodium bicarbonate and wherein the mixture of the gelatin solution and the sodium bicarbonate has a pH below 7.

Optionally said cross-linkable protein comprises gelatin in the form of a gelatin solution, comprising freeze-drying in a temperature and pressure range, optionally with aeration so as to foam the solution and form a dried porous layer of gelatin.

Another method of creating a porous sponge-like structure featuring a dry mixture of gelatin-enzyme is using the freeze-drying method but optionally without such aeration.

In this method, first, the homogenous gelatin solution is cooled rapidly, transforming it to a bi-phase solid which contains a gelatin rich continuous phase and an ice phase concentrated in secluded spots. Second, by reducing pressure in the freeze-drying chamber, the water in the solid undergoes sublimation, leaving pores in place where the ice phase was concentrated before.

Without wishing to be limited to a single range, in the freeze-drying process, it was shown that a porous flexible dry foam can be obtained with the same ratio of gelatin to mTG enzyme (80 U/g gelatin), but without physical aeration. The density of the dry foam is comparable to the one prepared by physical aeration, for example with N₂ gas. Accordingly, adhesion forces tested in-vitro were also comparable.

Optionally the method further comprises producing a gelatin layer by mixing a gelatin solution with said enzyme, said enzyme comprising transglutaminase, to form a foamed gelatin solution; wherein said method further comprises lyophilizing the foamed gelatin solution to form a lyophilized foamed gelatin solution and adding said lyophilized foamed gelatin solution to said product.

Optionally said transglutaminase is added to said gelatin solution prior to said mixing or during said mixing. Optionally said transglutaminase is added to said gelatin solution through continuous streaming during mixing.

Optionally the method further comprises cooling said foamed gelatin solution before said lyophilizing is performed. Optionally the method further comprises foaming a gelatin solution to form a foamed gelatin solution; drying the foamed gelatin solution to form said dried foamed gelatin solution; and adding said transglutaminase in a solution to said dried foamed gelatin solution to form an enzyme containing foam.

Optionally said transglutaminase to said dried solution comprises one or more of spraying an enzyme solution onto dry gelatin matrix surface; injecting an enzyme solution into the gelatin matrix through needles or matrix of needles; submersing dry gelatin matrix into an enzyme-containing solvent mixture; and/or dispensing enzyme-containing solvent mixture onto dry gelatin matrix.

Optionally the method further comprises drying said enzyme containing foam.

Optionally said drying said enzyme containing foam comprises one or more of air drying, vacuum drying, lyophilization and/or heat drying.

Optionally said drying occurs at a plurality of temperatures ranging from -40 C to 65 C. Optionally drying occurs at a temperature of up to 30 C. Optionally said drying occurs at a temperature of up to 20 C. Optionally said drying occurs at a plurality of temperatures ranging from 0 C to 20 C.

Optionally the mesh based composition comprises a plurality of gelatin layers and wherein optionally each of said gelatin layers has a different density of gelatin. Optionally at least one gelatin layer comprises a percentage of gelatin in solution of from about 1% w/w to about 15% w/w. Optionally at least one gelatin layer comprises a percentage of gelatin of from about 2.5% w/w to about 10% w/w. Optionally at least one gelatin layer comprises a percentage of gelatin of at least about 5% w/w.

Optionally at least one gelatin layer comprises a lubricant. Optionally said lubricant comprises glycerol. Optionally said glycerol is present in an amount of from 0.1% to 10%. Optionally said glycerol is present in an amount of from 2% to 6%.

According to at least some embodiments of the present invention, there is provided a mesh based composition comprising a cross-linkable porous protein matrix and a non blood-derived enzyme which induces cross-linking of the cross-linkable protein, wherein matrix density is in range of 5-100 mg/cm³. Optionally said density is in a range of 40-70 mg/cm³. Optionally the product has a total moisture content of less than 30%, a total moisture content of less than 20% or a total moisture content of less than 10%. Optionally such a mesh-based composition is useful for a variety of applications, including without limitation as a hemostatic dressing, tissue adhesive or wound closure.

Optionally a ratio of enzyme to matrix is from 0.05 to 5 mg enzyme/cm³ matrix. Optionally said ratio is 0.5 to 2.5 mg enzyme/cm³ matrix.

Optionally the product is sterilized to a sterility assurance level of 10^6 through exposure to electron beam radiation. Optionally the radiation dosage is in the range of 10-50 kGy. Optionally the radiation dosage is in the range of 20-40 kGy.

Optionally the product is sterilized to a sterility assurance level of 10-6 through exposure to ethylene oxide gas.

Optionally the product further comprises a radioprotectant selected from the group consisting of Ascorbate, Benzyl alcohol, Benzyl benzoate, Butylated Hydroxyanisole (BHA), Chlorobutanol, Cysteine, Mannitol, Methyl paraben, Niacinamide, Phenol, Propylene glycol, Propyl gallate, Propyl paraben, Sodium bisulfate, Sodium metabisulfite, Sodium salicylate, Sodium thiosulfate, Tocopherol, Trehalose.

Optionally the product further comprises a buffer optionally selected from the group including Sodium Acetate, HEPES, Sodium Citrate, Sodium Benzoate.

Optionally the product further comprises one or more foaming stabilizers, optionally selected from the group consisting of Ionic surfactants (i.e. SDS), Hydroxyl Propyl Methyl Cellulose, Hyaluronic Acid, Glycine, Dextran.

Optionally a plurality of discrete enzyme-containing protein matrix segments together form a single product. Optionally each segment is of diameter in range of 0.1 to 10 cm. Optionally each segment is of diameter in range of 1-5 cm.

**Table 2 showing synthetic surgical meshes that can be used as a component it the product described above**

| Commercial Mesh Name | Material | Manufacturer | |
|---|---|---|---|
| 1. | VICRYL ^{™} Woven Mesh | Polyglactin 910 (Polyglycolic Acid) | Ethicon (Somerville, NJ) |
| 2. | PROLENE ^{™} 3D Patch Polypropylene Mesh | Polypropylene | Ethicon |
| 3. | PROLENE ^{™} Polypropylene Mesh | Polypropylene | Ethicon |
| 4. | PROLENE ^{™} Polypropylene Hernia System | Polypropylene | Ethicon |
| 5. | MERSILENE ^{™} Polyester Fiber Mesh | Polyethylene Terephthalate | Ethicon |
| 6. | ULTRAPRO ^{™} Partially Absorbable Lightweight Mesh | Monocryl (Poliglecaprone 25) and Polypropylene | Ethicon |
| 7. | ULTRAPRO ^{™} Plug | Monocryl (Poliglecaprone 25) and Polypropylene | Ethicon |
| 8. | ULTRAPRO ^{™} Hernia System | Monocryl (Poliglecaprone 25) and Polypropylene | Ethicon |
| 9. | PVP ^{™} Device | Oxidized Regenerated Cellulose (ORC) and Polypropylene | Ethicon |
| 10. | PROCEED ^{™} Surgical Mesh | Oxidized regenerated cellulose (ORC) and Polypropylene | Ethicon |
| 11. | Parietex ^{™} Composite (PCO) Mesh | Macroporous Polyester, with a Three Dimensional Weave Material with resorbable collagen film | Covidien (Mansfield, MA) |
| 12. | Parietex ^{™} composite open skirt (PCO OS) mesh | Macroporous Polyester, with a three Dimensional Weave Material with resorbable collagen film | Covidien |
| 13. | Parietex ^{™} Composite (PCO) Parastomal | Macroporous Polyester, monofilament material | Covidien |
| | mesh | | |
| 14. | Parietex ^{™} Composite (PCO) Hiatal mesh | Macroporous Polyester, with a three Dimensional Weave Material with resorbable collagen film | Covidien |
| 15. | Parietex ^{™} anatomical mesh | Macroporous Polyester, 2D weave with 3D weave | Covidien |
| 16. | Parietex ^{™} Folding mesh | Macroporous Polyester | Covidien |
| 17. | Parietex Easegrip ^{™} mesh | Polyester, with a combination of two and three Dimensional Weave Material | Covidien |
| 18. | Parietex ^{™} lightweight monofilament mesh | Monofilament knit, macroporous polyester | Covidien |
| 19. | Parietex ^{™} Flat sheet mesh | Polyester, with both two and three Dimensional Weave options | Covidien |
| 20. | Surgipro ^{™} Flat Sheet mesh | Polypropylene | Covidien |
| 21. | PERFIX ^{™} Light Plug | Monofilament Polypropylene | Davol (Bard) (Warwick, RI) |
| 22. | PerFix ^{™} Plug | Monofilament Polypropylene | Davol (Bard) |
| 23. | Kugel ^{™} Patch | Monofilament Polypropylene | Davol (Bard) |
| 24. | 3DMax ^{™} Light Mesh | Monofilament Polypropylene | Davol (Bard) |
| 25. | Bard ^{™} Soft Mesh | Large pore monofilament polypropylene | Davol (Bard) |
| 26. | Bard ^{™} Mesh | Monofilament Polypropylene | Davol (Bard) |
| 27. | Bard ^{™} Visilex ^{™} Mesh | Monofilament Polypropylene | Davol (Bard) |
| 28. | Ventrio ^{™} Hernia Patch | Monofilament Polypropylene and polydioxanone, with Submicronic ePTFE side | Davol (Bard) |
| 29. | Composix ^{™} L/P Mesh | Low profile polypropylene Bard Soft Mesh and sub-micronic ePTFE side | Davol (Bard) |
| 30. | Composix ^{™} E/X | Polypropylene Bard Soft Mesh and sub-micronic ePTFE side | Davol (Bard) |
| 31. | Composix ^{™} Kugel ^{™} Patch | Self-expanding polypropylene/ePTFE mesh | Davol (Bard) |
| 32. | Dulex ^{™} Mesh | Dual-sided ePTFE mesh | Davol (Bard) |
| 33. | VENTRALEX ^{™} Hernia Patch | Self-expanding polypropylene and ePTFE | Davol (Bard) |
| 34. | Sepramesh ^{™} IP Composite | Polypropylene mesh with a hydrogel safety coating | Davol (Bard) |
| 35. | C-QUR ^{™} V-Patch | Polypropylene mesh with an all natural, pharmaceutical grade Atrium Omega 3 fatty acid | Atrium (Hudson, NH) |
| 36. | C-QUR ^{™} Mesh | Polypropylene mesh with an all natural, pharmaceutical grade Omega 3 fatty acid | Atrium |
| 37. | C-QUR Lite ^{™} Mesh | Polypropylene mesh with a thin, 30 day omega 3 fatty acid | Atrium |
| 38. | C-QUR Edge ^{™} | Bioabsorbable Oil (O3FA) Coated mesh features a reinforced edge design | Atrium |
| 39. | ProLoop ^{™} Mesh | Non-absorbable, lightweight, pre-formed, three-dimensional plug constructed of knitted rows of monofilament polypropylene with multiple protruding monofilament loops | Atrium |
| 40. | ProLite ^{™} Mesh | Polypropylene Mesh | Atrium |
| 41. | ProLite ^{™} Ultra ^{™} Mesh | Thin wall polypropylene mesh | Atrium |
| 42. | BIO-A ^{™} Tissue Reinforcement | Polyglycolic acid:Trimethylene carbonate (PGA:TMC) fibers form a non-woven web with open, highly interconnected pores | Gore Medical (Flagstaff, AZ) |
| 43. | DUALMESH ^{™} PLUS Biomaterial | Two-surface hernia repair material with antimicrobial technology | Gore |
| 44. | DUALMESH ^{™} Biomaterial | ePTFE material that offers two-surface design intended for minimizing tissue | Gore |
| | | attachment along another surface. | |
| 45. | MYCROMESH ^{™} Biomaterial | Microporous node and fibril structure with regularly spaced macropores. | Gore |
| 46. | MYCROMESH ^{™} PLUS Biomaterial | Includes antimicrobial technology | Gore |
| 47. | GORE-TEX ^{™} Soft Tissue Patch | Expanded polytetrafluoroethylene (ePTFE) | Gore |
| 48. | BIO-A ^{™} Hernia Plug | Porous fibrous structure composed of synthetic copolymer | Gore |
| 49. | INFINIT ^{™} Mesh | 100% monofilament PTFE, large pore knitted surgical mesh | Gore |

**Table 3 showing biological surgical meshes**

| Commercial Mesh Name | Material | Manufacturer | |
|---|---|---|---|
| 1. | FLEXHD ^{™} Acellular Hydrated Dermis | Acellular human skin | Ethicon |
| 2. | Permacol ^{™} Biologic Implant | Derived from porcine dermal collagen | Covidien |
| 3. | XENMATRIX ^{™} Surgical Graft | Non-crosslinked collagen matrix | Davol (Bard) |
| 4. | COLLAMEND ^{™} FM Implants | All-natural porcine collagen | Davol (Bard) |
| 5. | AlloMax ^{™} Surgical Graft | All-natural biologic implant derived from human dermal collagen. | Davol (Bard) |
| 6. | Biodesign ^{™} (Surgisis ^{™}) Hernia Graft | Dry, acellular porcine small intestinal submucosa | Cook Biotech (Lafayette, IN) |
| 7. | Biodesign ^{™} (Surgisis ^{™}) Hiatal Hernia Graft | Dry, acellular porcine small intestinal submucosa | Cook Biotech |
| 8. | Biodesign ^{™} (Surgisis ^{™}) Inguinal Hernia Graft | Dry, acellular porcine small intestinal submucosa | Cook Biotech |
| 9. | Biodesign ^{™} (Surgisis ^{™}) | Dry, acellular porcine small | Cook Biotech |
| | Umbilical Hernia Graft | intestinal submucosa | |
| 10. | Biodesign ^{™} (Surgisis ^{™}) Abdominal Lock Graft | Dry, acellular porcine small intestinal submucosa | Cook Biotech |
| 11. | Biodesign ^{™} (Surgisis ^{™}) 8-Layer Tissue Graft | Dry, acellular porcine small intestinal submucosa | Cook Biotech |
| 12. | Strattice ^{™} Reconstructive Tissue Matrix | Decellularized porcine skin | LifeCell - Genzyme Corp (Branchburg, NJ) |
| 13. | AlloDerm ^{™} | Decellularized human cadaver skin | LifeCell |

### Copolymers of Crosslinkable Protein/Polypeptide

According to some embodiments, polyethylene glycol (PEG), also known as poly(ethylene oxide) (PEO) or polyoxyethylene (POE), is added to the protein/polypeptide or crosslinker solution as a copolymer, to improve one or more properties of the composition, for example (and without limitation) to increase the flexibility of the composition or to shield from the body's immune response to the protein-crosslinker composition. PEG is available over a wide range of molecular weights from 300 Da to 10 MDa and may be a liquid or low-melting solid, depending on the molecular weights.

Different forms of chemically-modified PEG are also available, depending on the initiator used for the polymerization process, the most common of which is a monofunctional methyl ether PEG (methoxypoly(ethylene glycol)). PEGs are also available with different geometries. Branched PEGs have 3 to 10 PEG chains emanating from a central core group. Star PEGs have 10 - 100 PEG chains emanating from a central core group. Comb PEGs have multiple PEG chains normally grafted to a polymer backbone. All of these types of PEGs should be considered useful in the present invention.

PEGs can be added to either the protein or crosslinker components of a protein-crosslinker composition. Preferentially, PEG is added at a dry weight ratio between 20:1 to 1:1, protein:PEG. PEG can be added to the protein component or crosslinker component through modification of the protein or crosslinker and/or modification of the PEG molecules. One example of such modification is the process known as PEGylation. PEGylation is the act of covalently coupling a PEG structure to another larger molecule. This process can be performed on either the protein or crosslinker molecules.

The gelatin PEGylation embodiment has, among its many advantages and without wishing to be limiting, the advantage that the PEG is part of the protein chain, therefore inducing changes in properties of the protein surface including but not limited to charge and hydrophilicity, as well as steric effects that are due to its bulkiness. The embodiment is described for example in US Patent No. 8,367,388, filed on June 18 2009, owned in common with the present application and having at least one inventor in common with the present application. As a result, the covalently attached PEG can have profound effects on intermolecular interactions between protein chains and in turn on physical gelation and crosslinker dependent crosslinking as well as on the mechanical properties of gels prepared by these methods .

The PEG molecules used in PEGylation are usually activated , meaning they react spontaneously with functional groups on the target protein. A non limiting example of PEGylation is using NHS ester derivatives of PEG. These activated PEG molecules react with primary amines on proteins to form amide bonds with the release of N-hydroxysuccinimide (NHS).

Other ways in which a protein can be modified is by reacting the primary amines found inside chains of lysine and at the amino termini of the protein chains. The modification may be by alkylation, succinylation, carbamylation, or by any other method of protein modification.

In a preferred embodiment, the crosslinkable protein/polypeptide is first reacted with activated PEG to create PEGylated protein. The PEGylated protein is purified from excess unreacted PEG and other reaction products by methods such as, but not limited to, dialysis, ultrafiltration, and gel filtration chromatography. The PEGylated protein can then be reacted with a crosslinker to form a crosslinked gel.

PEGs can also optionally be added through the use of PEG amine as a substrate for a crosslinker that targets amine groups. The crosslinker crosslinks the PEG molecule through its terminal amine group to crosslinker substrates on the protein molecule, thus competing with the natural amine groups on the protein.

PEG amines comprise PEG that has been bound to amine-functional groups. These are commercially available in all types of PEG geometries. Sources of amine-functional PEG products include NOF (Japan), Nanocs (New York, NY) and Pierce Biotechnology (Rockford, IL).

In all approaches of incorporating PEG, the number of natural substrates available for crosslinking is reduced, resulting in reduced cross-linking. This may affect the mechanical properties of the crosslinked gel, for example optionally allowing it to become less rigid and more flexible. In addition, and without wishing to be limited by a single hypothesis, the PEG molecule itself may act as a plasticizer and further contribute to the flexibility of the resulting gel.

According to a preferred embodiment, the PEG amine comprises active lysine amino acids.

According to another embodiment, of the present invention, Polyvinyl Alcohol (PVA) is added to a gelatin or mTG solution as a copolymer to increase the flexibility or adhesiveness of a protein-crosslinker composition. PVA is a water-soluble synthetic polymer with high tensile strength and flexibility. In a high humidity environment, such as inside the body, PVA will absorb water. The water, which acts as a plasticizer, can then reduce the tensile strength of the PVA, but increase its elongation.

According to some embodiments, the copolymer comprises PVA-amine. When the amine-targeting crosslinker is added to the solution, both the protein and PVA-amine will act as substrates and a protein-PVA copolymer will be formed with better flexibility than a comparable cross-linked protein polymer.

A non-limiting example of a process that can be used for producing amine functional derivatives of poly (vinyl alcohol) is described in US Patent 6107401.

Another non-limiting example of a process that can be used for producing an amine copolymer of PVA is described in US Patent 4931501 where poly(vinyl alcohol) is reacted with an amino-aldehyde dialkyl acetal.

A process of synthesizing amine-modified poly(vinyl alcohol)s by a two-step process using carbonyl diimidazole activated diamines to produce PVAs with different degrees of amine substitution has also previously been described (Wittman M, et al. Biophysical and Transfection Studies of an Amine-Modified Poly(vinyl alcohol) for Gene Delivery. Bioconjugate Chem., 16 (6), 1390 - 1398 , 2005), as another non-limiting example.

According to some embodiments of the present invention, there is provided a composition comprising gelatin, transglutaminase and a calcium crosslinkable alginate matrix. Optionally said calcium crosslinkable alginate matrix is added in a weight ratio of between 1 to 30% weight per weight according to the weight of the gelatin, and preferably in the ratio of 5 to 20%.

Under the pH used in an exemplary embodiment (pH 3.8), a precipitate was formed. When such a precipitate is formed, it may optionally be dissolved by addition of a suitable salt in a suitable amount, such as NaCl for example. In this non-limiting example and without wishing to be limited by a single hypothesis, alginate may have precipitated alone, and/or gelatin and alginate may have formed a polyelectrolyte complex which precipitated from the solution. As noted previously, it was found out that addition of 12gr/L or 0.2M NaCl dissolved the precipitate and clarified the solution.

Optionally gelatin, transglutaminase, calcium crosslinkable alginate matrix, a suitable salt such as NaCl in a suitable amount, optionally with additional additives, may be dissolved homogenically in solution. Mesh based compositions may be prepared in the same methods described above from said solution.

According to some embodiments of the present invention, there is provided a composition comprising gelatin, transglutaminase and Chitosan.

Optionally said Chitosan is added in a weight ratio of between 1 to 100% to the gelatin weight, more preferably in the ratio of 20 to 100%.

### Surfactants

According to some embodiments of the present invention, one or more biocompatible surfactants are added to the solution of cross-linkable protein or polypeptide, for example in order to reduce the surface tension of that solution.

Surfactants are wetting agents that lower the surface tension of a liquid, allowing easier spreading, and lower the interfacial tension between two liquids. Lower surface tension facilitates easier handling of a solution of a cross-linkable peptide as it is easier to pass through an applicator, and easier to mix with a solution of a cross-linking material. Surfactants can also lower the viscosity of the solution. Additionally, lowering the surface tension of a gelatin solution has great utility when a gelatin solution is lyophilized either alone or together with a mTG solution, as it can prevent the formation of a film on the top layer of the dried gelatin. Such a film inhibits the reconstitution of lyophilized gelatin into a homogenous solution.

Non-limiting examples of biocompatible surfactants useful in context of the present invention are polysorbate 20 (Tween^{™} 20), polyoxyethyleneglycol dodecyl ether (Brij^{™} 35), polyoxyethylene-polyoxypropylene block copolymer (Pluronic^{™} F-68), sodium lauryl sulfate (SLS) or sodium dodecyl sulfate (SDS), sodium laureth sulfate or sodium lauryl ether sulfate (SLES), poloxamers or poloxamines, alkyl polyglucosides, fatty alchohols, fatty acid salts, cocamide monoethanolamine, and cocamide diethanolamine.

Surfactants may be used also as plasticizers. Tween80 for example has been shown to reduce the glass transition point (T_{g}) of several hydrophilic polymers. The presence of the smaller molecules of Tween80 within the polymer were thought to dilute and weaken the cohesive interactions between the polymers chains. This reduced the friction and entanglement by increasing the free volume in the polymer matrix. (Ghebremeskel et al, 2006, International Journal of Pharmaceutics 328:119-129).

In a preferred embodiment of the present invention, one or more surfactants are used as a plasticizer to improve the elasticity of the crosslinked composition, particularly as it stiffens over time.

In another optional embodiment, one or more surfactants are combined with another plasticizer from the plasticizers listed above as relevant to the present invention. Rodriguez et al (Food Research International 39 (2006) 840-6) demonstrated a synergistic effect between a plasticizer (glycerol) and surfactants (Tween20, Span 80, Lecithin) on increasing the elasticity of non-crosslinked dry gelatin films.

Preferentially, surfactants are added to a gelatin solution at a weight ratio of 0.1-5% of the dry weight of gelatin in the solution. Alternatively, surfactants are added to a gelatin solution at a concentration approximately equal to the critical micelle concentration (CMC) of that particular surfactant in solution. The CMC of each surfactant varies and is dependant on the ionic concentration of the solution into which the surfactant is dissolved.

### Configurations of Lyophilized Product

In an embodiment of the present invention, a dried composition is formed wherein the dry crosslinker material is thoroughly dispersed through a lyophilized composition of cross-linkable protein or polypeptide.

In an embodiment of the present invention, a dried or frozen composition is formed wherein the cross-linkable protein or polypeptide is thoroughly mixed with the non-toxic cross-linker to form a homogenous solution and the temperature of the solutions is reduced immediately to prevent completion of the cross-linking process. The mixed composition is then either frozen or frozen and dried to form a novel, uniform composition.

In another embodiment, the protein is gelatin and a non-crosslinked gelatin foam is lyophilized prior to dispersal of crosslinker throughout such a porous foam.

In another embodiment, dry crosslinker material is added to the gelatin foam such that the crosslinker does not dissolve into the foam (ie no crosslinking activity is observed prior to lyophilization).

It was surprisingly found that a reconstitutable foam could optionally be formed from a gelatin solution that was sufficiently stabile so as to allow for the lyophilization of the gelatin in foam form without the addition of any stabilizing or crosslinking agents.

In a preferred, illustrative embodiment of the present invention for forming such a foam, a gelatin solution is prepared and held at a temperature where it is in liquid form. The gelatin solution is then subjected to an extended and preferably continuous foaming process while it is cooled to a temperature below its sol-gel transition point.

The concentration of gelatin solution is preferably in the range of 0.5% - 20% w/w, more preferably 5-10% w/w.

The initial temperature of the gelatin solution is 30°C-70°C, preferably 30°C -50°C, and more preferably 35°C -45°C. The environmental temperature during the foaming process is 0°C-25°C, preferably 15°C-25°C. Non-limiting examples of foaming processes include stirring, mixing, blending, and injection of a gas.

Preferably, the foaming process includes stirring or mixing.

One or more foaming techniques may optionally be used in the foaming process. Alternatively, one foaming technique may optionally be used multiple times under different conditions: for example, gentle stirring to generate a low level of foam following by vigorous stirring to achieve maximal aeration in the gelatin foam.

In an optional embodiment, upon the completion of foaming, the gelatin foam is preferably transferred to a vessel that had been cooled to a temperature lower than the temperature of the gelatin foam upon completion of the foaming process.

In another embodiment, the gelatin foam is optionally and preferably rapidly cooled immediately upon completion of foaming process. A non-limiting example of rapid cooling is exposing gelatin foam to liquid nitrogen immediately after the foaming process.

In a preferred embodiment, the dry gelatin foam contains less than about 15% moisture. In a more preferred embodiment, the dry gelatin foam contains less than about 10% moisture.

In another embodiment, the gelatin foam is optionally not further stabilized by cooling or other method upon the completion of foaming such that the foam partially collapses resulting in the formation of a denser layer of gelatin foam on the bottom of the foam. For example, optionally a period of time elapses before such stabilization is performed, for example and without limitation up to 10 minutes. If stabilization comprises cooling, optionally such cooling is delayed by between 2 to 10 minutes after foaming.

In an embodiment of the above, the denser layer optionally comprises less than about 50% of the thickness of the lyophilized gelatin composition, preferably less than about 35%, and more preferably less than about 20%.

Density as used herein refers to an increase in the weight of gelatin per volume of lyophilized composition. Such an increase can optionally be as little as 5% but is preferably greater than about 10% and more preferably greater than about 20%.

Without wishing to be limited to a single hypothesis or to a closed list, it is believed that such a dense layer of gelatin foam provides mechanical strength to the lyophilized gelatin composition without affecting the reconstitution profile of the top part of the dry composition.

### Mesh-based Composition Preparation Methods

Different suitable preparation methods may optionally be used for preparation of the mesh-based composition (not defined by the scope of the claims). Various exemplary stages are given below, which may optionally be differently configured and recombined. Each such exemplary stage may optionally be performed according to different methods, each of which may optionally be combined with any other preparation method as described herein. The stages are given in the order in which they are optionally performed.

Backing (non-adhesive component) application may optionally comprise blade coating, i.e. a gap controller which extrudes the material, thus determining the wet thickness of the applied coating. Additionally or alternatively, such backing application may optionally comprise one or more of air knife coating, spraying, silk printing or any other relevant printing process, spin coating, dip coating, curtain coating, solution casting, any other suitable method for coating layers of polymer dispersions /solutions, and/or melt extrusion (no solvent involved, only heating the polymer).

Creating the porous structure for the adhesive matrix may optionally comprise (physical) gas foaming, which involves introducing pressurized inert gas (e.g. nitrogen) to the gelatin solution. Additionally or alternatively, such creation of the porous structure may optionally comprise one or more of (physical) emulsion freeze drying, (chemical) solvent casting/particulate leaching, (physical) high pressure processing with supercritical gas, e.g. co₂, (physical and/or chemical) gas foaming/particulate leaching, e.g. with dispersed ammonium bicarbonate salt particles, thermally induced phase separation, electrospinning, and/or rapid prototyping (for example with a 3D printer).

Next Mixing gelatin and enzyme is performed, for example after gelatin solution foaming, using static mixers (mixing element is rotating by the movement of the material through it). Enzyme solution is introduced after gelatin solution is foamed. Various methods may optionally be performed, such as for example mixing gelatin and enzyme together in the same solution as first step, for example and without limitation mixing in a form of an oil in water or water in oil emulsion and/or mixing encapsulated enzyme micro-particulates as a dispersion in a gelatin continuous phase. In another exemplary method, the enzyme is introduced in a later stage, for example and without limitation deploying enzyme dry particulates over the pre-dried gelatin matrix, and/or layer by layer application of alternating gelatin and enzyme layers.

Next adhesive layer (gelatin-enzyme foamed solution) application is performed, for example with blade coating, i.e. a gap controller which extrudes the material, thus determining the wet thickness of the applied coating. Other exemplary methods include without limitation air knife coating, spraying, silk printing or any other relevant printing process, spin coating, dip coating, curtain coating, solution casting, a suitable method for coating layers of polymer dispersions /solutions, injection molding and/or molding (casting) .

Polypropylene mesh positioning may optionally be performed in various ways. For example and without limitation, optionally adhesive layer application is performed in two steps, in which the polypropylene mesh is positioned over the first adhesive layer, then second adhesive layer covers it. Optionally the mesh is positioned before or after foam application.

Attachment of adhesive and backing layers may optionally be performed through direct application of the adhesive foamed solution over the already dry backing. Additionally or alternatively, such attachment may optionally performed through one or more of drying the two films separately and then attaching them through a suitable method, for example and without limitation by using an adhesive (film transfer method) or by a lamination process. Optionally the backing is applied on top of the already dry adhesive layer, for example and without limitation by air knife coating, spraying, silk printing or any other relevant printing process, spin coating, curtain coating, any other methods known for coating layers of polymer dispersions /solution, and/or hot-melt extrusion.

Drying of the adhesive layer may optionally be performed through lyophilization (freeze-drying) at -20oC. Such drying may optionally also be performed, additionally or alternatively through one or more of hot air drying, vacuum drying, or by keeping the product frozen until application.

### Mesh Device Dimensions

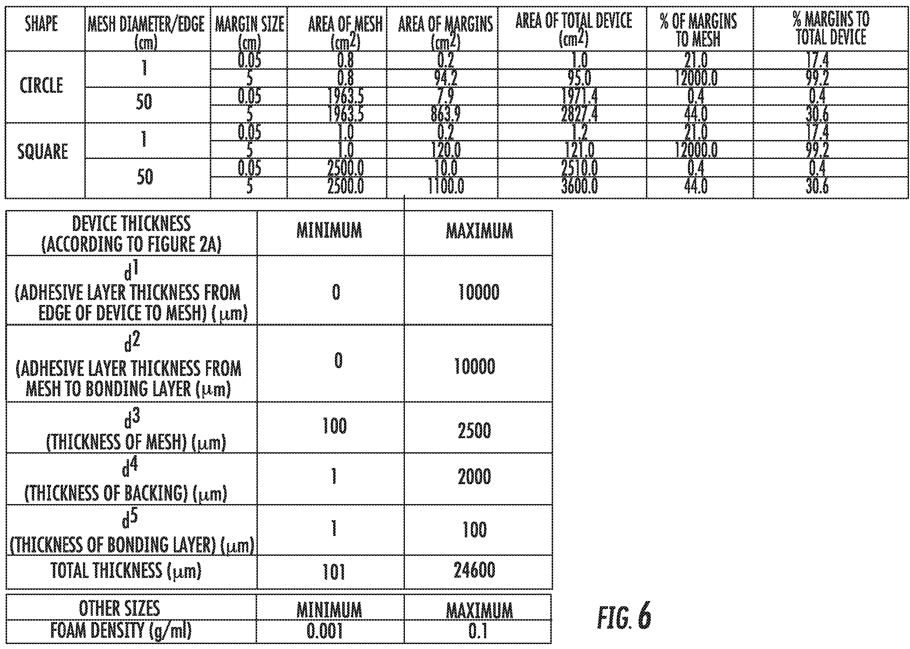

The mesh-based composition may also optionally be described as a mesh device with particular dimensions. Non-limiting examples of such dimensions are provided below and in Figure 6.

The dimensions may optionally vary for example according to the shape of the device, for example as a rectangle, optionally including a square, or as a rounded shape, optionally include an ellipse or a circle. The diameter (or distance from edge to edge for a non-rounded shape) may optionally be in a range of from 0.5 cm to 60 cm. Non-limiting examples are given as 1 cm and 50 cm in Figure 6. However, optionally these dimensions only relate to the mesh size but not the size of the total device. For such an implementation, the size of the total device is given below.

The area of the mesh in cm squared optionally ranges from 0.5 cm² to 3600 cm². Non-limiting examples of the area of the mesh are given as 0.8 cm² for a 1 square cm device if rounded, or 1 cm² if not rounded. The area of the mesh is not necessarily the total area of the device according to those implementations in which the above dimensions for the device do not include the margin for example and/or otherwise do not relate to the total dimensions of the device.

The margin size is the section of the device that extends beyond the mesh, which = = comprises an adhesive. The margin size may optionally be in a range of from 0.01 to 10 cm. Non-limiting examples are given as 0.1 cm and 5 cm in Figure 6. The margin size as a percentage of the diameter or edge to edge distance optionally ranges from 0.1% to 10%.

The area of the margin is optionally from 0.0001 cm² to 1200 cm², but is preferably from 0.1 cm² to 900 cm² for round shaped devices, and 0.1 cm² to 1100 cm² for square shaped devices.

The area of the total device, when different from the dimensions given above, optionally ranges from 0.5 cm² to 3000 cm² for round shaped devices, and from 0.5 cm² to 4000 cm² for square shaped devices.

The margin area as a percentage of the mesh area optionally ranges from 0.1 cm² to 14,000 cm². The margin area as a percentage of the total device area optionally ranges from 0.1 % to 100 %.

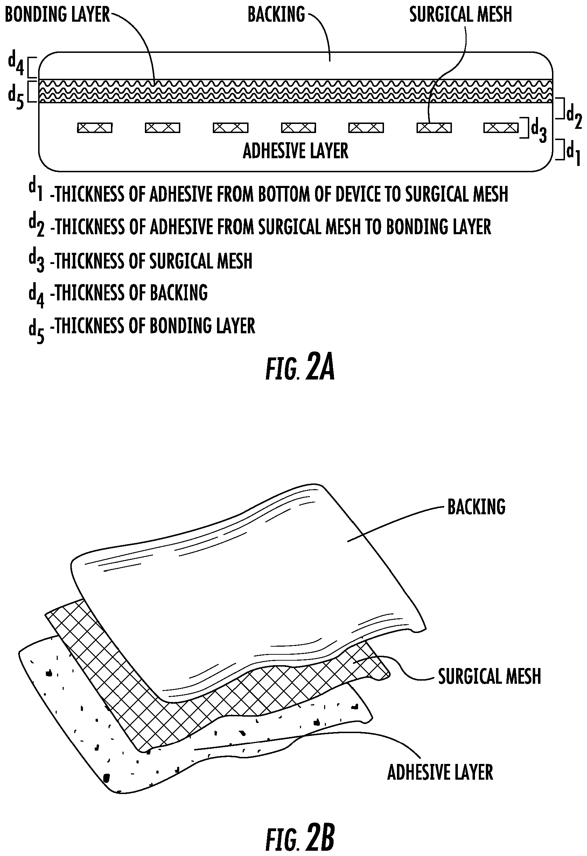

The dimensions of the thickness of different parameters of the device, according to the symbols given in Figure 2A, optionally range for d1 (adhesive layer thickness from edge of device to mesh) (µm) from 0 to 10000; for d2 (adhesive layer thickness from mesh to bonding layer) (µm) from 0 to 10000; for d3 (thickness of foam enclosed within the mesh) (µm) from 100 to 2500; for d4 (thickness of backing) (µm) from 1 to 2000; and for d5 (thickness of bonding layer) (µm) from 1 to 100. The total thickness (µm) ranges from 101 to 24600.

The ratios (as a percentage) of the dimensions of the thickness of different parameters of the device optionally range for the % of backing thickness to foam thickness from 0.004 to 2000.000; and for the % of backing to total thickness, from 0.004 to 1980.198.

The foam density (g/ml) optionally ranges from 0.001 to 0.1.

Optionally all dimensions, percentages and other parameter values described herein may be increased or decreased by 10%, and are still considered to be within the present invention as described herein.

### EXAMPLES

Reference is now made to the following examples, which together with the above description, illustrate some embodiments of the invention in a non limiting fashion.

### Example 1:

As shown herein, various exemplary implementations of the structure featuring the mesh-based composition are possible and are considered to fall within the present invention.

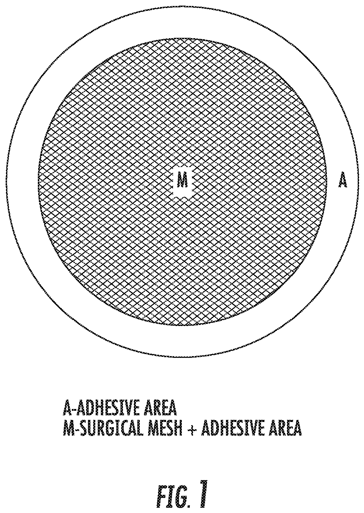

Fig. 1 shows a top view of the device design, including a section of adhesive covered mesh and a section of only adhesive surrounding the mesh-adhesive section.

Fig. 2A shows a side view of the device portraying an optional placement of the mesh within the adhesive, in a way that the adhesive surrounds the mesh in all directions. Figure 2B shows an exploded view of the layers.

A non-limiting example of mesh materials and the associated composition are provided in Table 4 below.

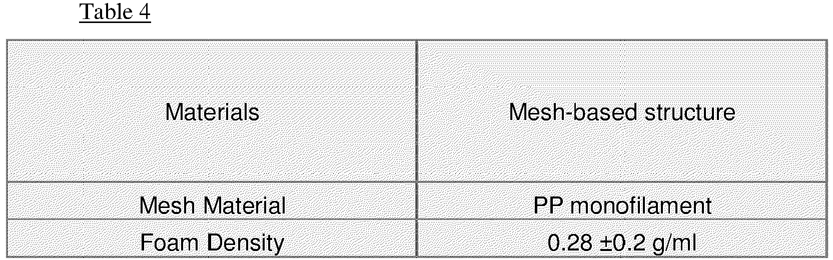

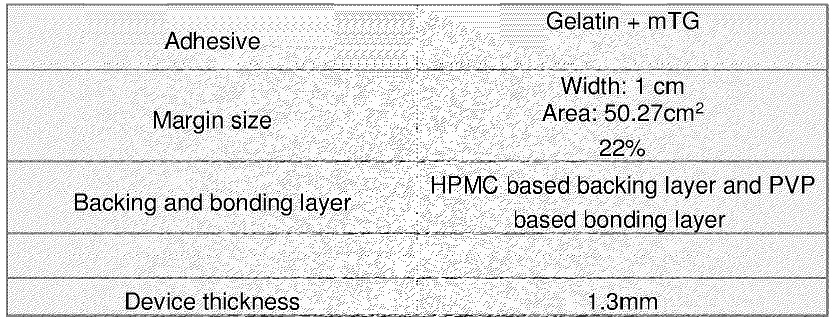

The adhesive may optionally be composed as described in US Patent No. 8,961,544 B2, = owned in common with the present application and having at least some inventors in common with the present application. Other materials and parameters as given above are as examples only, without any intention of being limiting.

### Example 2:

The tackiness of various substrates to moist latex glove was tested using a vertical electromechanocal testing system (Instron, UK). Two types of backing were used, crosslinked (CL) gelatin and HPMC. The backings covered a layer of a non-crosslinked foamed gelatin. A flat attachment was connected to the Instron head to which a latex glove was attached tightly. The latex glove was dipped in 0.9% saline prior to attachment to the Instron. A sample of foamed adhesive and backing was connected to the bottom flat plate with double sided tape. The Instron head was compressed perpendicularly, until a force of 0.3-0.9 N was achieved. The force was held for a second and extension was performed while extension load was measured by the 50 N load cell.

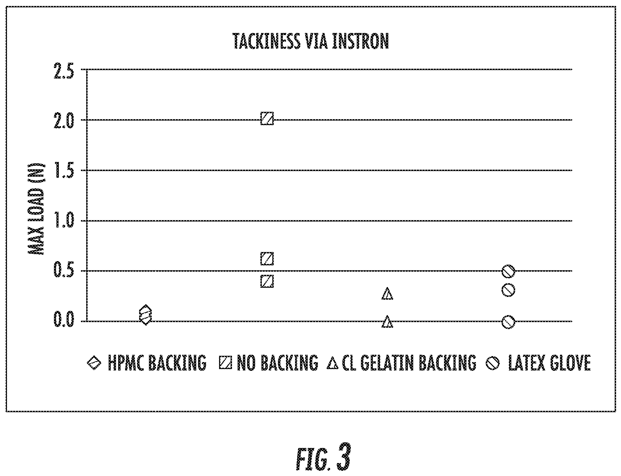

The data clearly shows that the backing decreases the tackiness of the foamed gelatin layer, and is not different than the tackiness between latex gloves to itself. The results are shown in Figure 3, which shows the measurement of relative tackiness of the various backing and composition combinations.

### Example 3

Backings were prepared as films by casting, and after drying were cut into 1 cm wide strips and placed into the holders of the vertical electromechanocal testing system (Instron, UK). The film length was 5 cm. Backing was set on bottom and upper holders, where the bottom holder was fixed, and the upper holder was connected to 50 N load cell. After this, tensile extension of the sample was performed. Extension rate was set to 0.5mm/s, while tensile stress and strain were measured.

The data shows that the HPMC backing becomes more elastic when plasticizers are added and that this effect is concentration dependent. PEG400 is a better plasticizer than sorbitol, when same amounts are compared. The results are shown in Table 5 below.

**Table 5 - effect of plasticizer on elasticity**

| Backing composition | % plasticizer | % elongation |
|---|---|---|
| 1% HPMC | 0 | 1.11 |
| 1% HPMC + 0.1% PEG400 | 9.1 | 3.49 |
| 1% HPMC + 0.3% PEG400 | 23 | 9.74 |
| 1% HPMC + 0.1% sorbitol | 9.1 | 1.87 |
| 1% HPMC + 0.3% sorbitol | 23 | 4.57 |

### Example 4

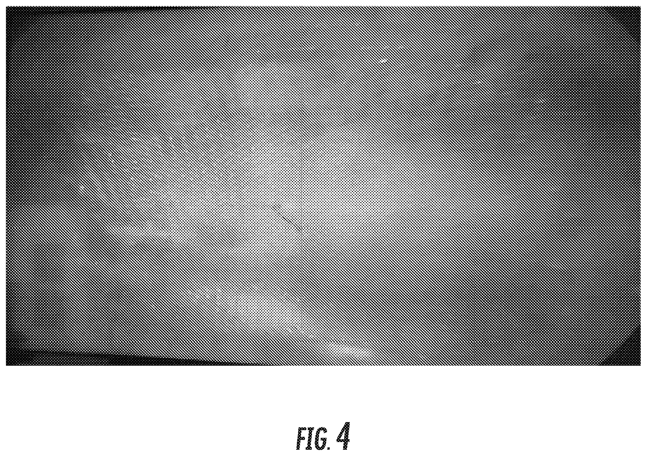

This example relates to the surrounding adhesive margin section (A in fig 1) and its performance in vivo; Figure 4 shows a lightweight mesh fixated to peritoneal tissue in vivo using the gelatin-mTG adhesive matrix. This image shows a mesh based device that was inserted laparoscopically into place and applied to the porcine peritoneum. The size of the mesh applied is 15x15cm of surgical mesh surrounded by adhesive and covered by a backing layer (HPMC (k100) + HPMC (k4)) and a bonding layer composed of Plasdone C17), plus 1 cm margins on the periphery which include only adhesive and backing (connected with the bonding layer), without mesh. These margins can be seen in the image as having a slightly different color than the center of the device and the tissue surrounding the surgical site. In this implantation, the mesh succesfully adhered to the tissue due to the adhesive layer and the backing prevented any tackiness to surgical tools.

### Example 5

This Example relates to non-limiting, exemplary methods of preparation of mesh based compositions.

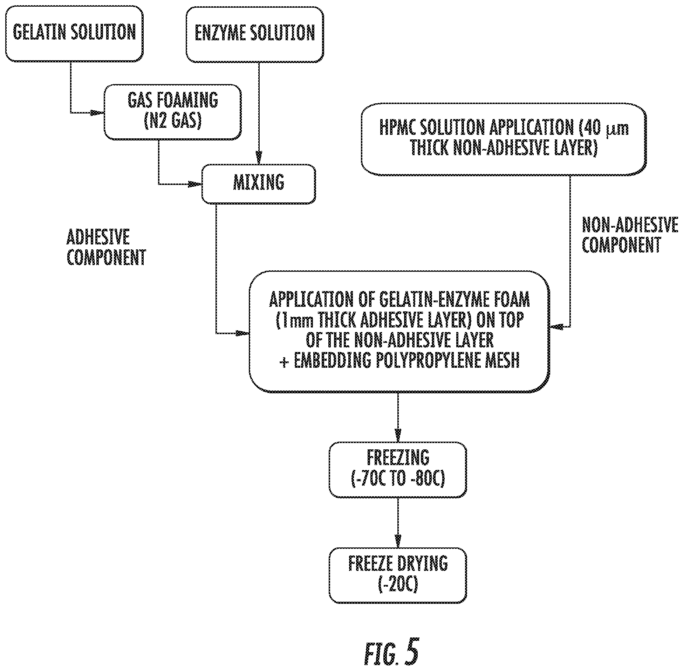

As shown on the left-hand branch of Figure 5, in blue, the adhesive composition is optionally prepared as follows. The gelatin solution is optionally foamed first with gas, preferably an inert gas such as nitrogen. Next the foamed solution is mixed with the enzyme solution. This adhesive composition is then optionally combined with the non-adhesive component, such as HPMC for example, by applying the adhesive layer on the non-adhesive layer, and then embedding the mesh. This mesh-composition combination is then optionally first frozen at -70 to -80 C, and then freeze dried at -20 to 15 C.

Table 6 relates to various optional and non-limiting methods of preparation at each stage of the process.

**Table 6 - process alternatives**

| **Stage in process** | **Example method** | **Other possible methods** | |
|---|---|---|---|
| Backing (non-adhesive component) application | Blade coating, i.e. a gap controller which extrudes the material, thus determining the wet thickness of the applied coating. | 1. Air knife coating. | |
| | | 2. Spraying. | |
| | | 3. Silk printing or any other relevant printing process for that matter. | |
| | | 4. Spin coating. | |
| | | 5. Dip coating. | |
| | | 6. Curtain coating. | |
| | | 7. Solution casting. | |
| | | 8. Any other methods known for coating layers of polymer dispersions /solutions. | |
| | | 9. Melt extrusion (no solvent involved, only heating the polymer). | |
| Creating the porous structure for the adhesive matrix | (Physical) Gas foaming - Introducing pressurized inert gas (e.g. Nitrogen) to the gelatin solution. | 1. (Physical) Emulsion freeze drying. | |
| | | 2. (chemical) Solvent casting/particulate leaching. | |
| | | 3. (Physical) High pressure processing with supercritical gas, e.g. CO2. | |
| | | 4. (Physical/chemical) Gas foaming/particulate leaching, e.g. with dispersed ammonium bicarbonate salt particles. | |
| | | 5. Thermally induced phase separation. | |
| | | 6. Electrospinning. | |
| | | 7. Rapid prototyping. | |
| Mixing gelatin and enzyme. | Mixing is performed after gelatin solution foaming, using static mixers (mixing element is rotating by the movement of the material through it). Enzyme solution is introduced after gelatin solution is foamed. | 1 Mixing gelatin and enzyme together in the same solution as first step, e.g.: | |
| | | | 1.1 Mixing in a form of an oil in water or water in oil emulsion. |
| | | | 1.2 Mixing encapsulated enzyme micro-particulates as a dispersion in a gelatin continuous phase. |
| | | 2 Introducing enzyme in later steps, e.g.: | |
| | | | 2.1 Deploying enzyme dry particulates over the pre-dried gelatin matrix. |
| | | | 2.2 Layer by layer application of alternating gelatin and enzyme layers. |
| Adhesive layer (gelatin-enzyme foamed solution) application | Blade coating, i.e. a gap controller which extrudes the material, thus determining the wet thickness of the applied coating. | 1. Air knife coating. | |
| | | 2. Spraying. | |
| | | 3. Silk printing or any other relevant printing process for that matter. | |
| | | 4. Spin coating. | |
| | | 5. Dip coating. | |
| | | 6. Curtain coating. | |
| | | 7. Solution casting. | |
| | | 8. Any other methods known for coating layers of polymer dispersions /solutions. | |
| | | 9. Injection molding. | |
| | | 10. Molding (casting). | |
| Polypropylene mesh positioning | Adhesive layer application is performed in two steps - the polypropylene mesh is positioned over the first adhesive layer, then second adhesive layer covers it. | 1. Positioning the mesh before foam application. | |
| | | 2. Positioning the mesh after foam application. | |
| Attachment of adhesive and backing layers | Direct application of the adhesive foamed solution over the already dry backing. | 1. Drying the two films separately and then attaching them e.g.: | |
| | | | 1.1 Using an adhesive (film transfer method). |
| | | | 1.2 By a lamination process. |
| | | 2. Application of the backing on top of the already dry adhesive layer, by e.g.: | |
| | | | 2.2 Air knife coating. |
| | | | 2.3 Spraying. |
| | | | 2.4 Silk printing or any other relevant printing process for that matter. |
| | | | 2.5 Spin coating. |
| | | | 2.6 Curtain coating. |
| | | | 2.7 Any other methods known for coating layers of polymer dispersions /solutions. |
| | | | 2.8 Hot-Melt extrusion. |
| Drying of the adhesive layer | Lyophilization (Freeze-drying) at -20°C. | 1. Hot air drying. | |
| | | 2. Vacuum drying. | |
| | | 3. None at all, keeping the product frozen until application. | |

Figure 6 provides some optional dimensions, minimum and maximum, for various patch shapes for patches that are prepared according to the above process. Figure 1 shows how the dimensions given relate to the product. In particular, the letter "A" shows the section where adhesive is present, while the letter "M" indicates that both the mesh and adhesive are present. The following dimensions are given in Figure 6 and are explained below:
d₁ - Thickness of adhesive from bottom of device to surgical mesh
d₂ - Thickness of adhesive from surgical mesh to bonding Layer
d₃ - Thickness of surgical mesh
d₄ - Thickness of backing
d₅ - Thickness of bonding layer

### EXAMPLE 6 - Inhibition of mTG enzyme in-process using acetic acid

The inhibition of the mTG enzyme throughout the "wet" part of the mesh preparation process through reduction in pH value helps prevent premature cross-linking of the gelatin solution.

### Methods:

### Preparation of gelatin solution - 9% w/w, (example for pH 3.8) :

12285g purified water was pre-heated to 40-45□c. 1350g gelatin was added to the vessel gradually, while stirring with coned over-head stirrer.

After full dissolution of the gelatin, 1365g 3M acetic acid (360g 100% glacial acetic acid (J.T Baker) mixed with 1640g PW) were added to the vessel while stirring for 5 minutes . The pH was tested with pH meter to be 3.8±0.5.

In the same way, gelatin solutions with pH 5.5, 4.0 and 3.5 were prepared. Purified mTG enzyme solution containing a sodium citrate buffer (preparation described below), 50 U/ml was added to contain 40 U/g gelatin.

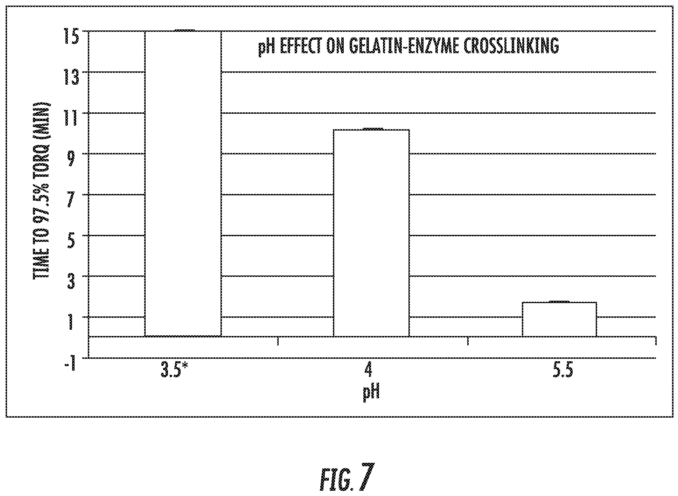

Figure 7 shows the effect of pH on mTG activity by viscometer test. 9% gelatin in pH 5.5 (native), 4 and 3.5 were mixed with 40u/ml enzyme solution at 37°c(2:1) . For pH 3.5 (indicated with an *), the test was stopped after 15 minutes as the viscosity didn't rise above 0.1% .

### EXAMPLE 7 - Methods for creation of a porous adhesion layer

### Physical aeration with pressurized gas

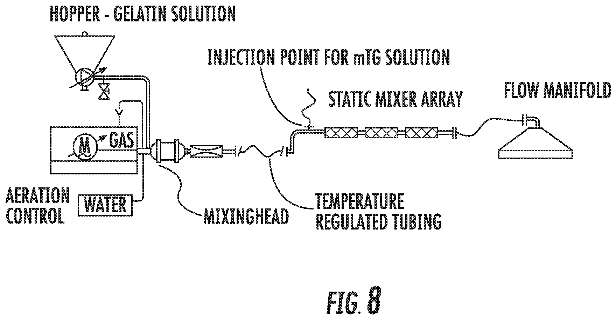

Figure 8 shows a schematic view of the aeration and mixing system, featuring a hopper into which the gelating solution is placed for aeration. Aeration occurs at the mixing head, followed by movement through temperature controlled tubing. After injection of the mTG solution, static mixing occurs. The material then flows out through a flow manifold.

### Methods:

Preparation of gelatin solution - 9% w/w water, pH=3.8, 15 kg; 12285g purified water was pre-heated to 40-45□c . 1350g gelatin was added to the vessel gradually, while stirring with coned over-head stirrer. After full dissolution of the gelatin, 1365g acetic acid were added to the vessel while stirring for 5 minutes. The pH was tested with pH meter to be 3.8±0.5.

### Preparation of Microbial transglutaminase solution

56.5g of concentrated purified mTG solution (885 U/ml) was mixed with 943.5g 20mM sodium citrate solution, to yield a diluted mTG solution (50 U/ml).

Acidified Gelatin solution was pumped to the aeration machine, in which pressurized nitrogen gas was introduced to the pressure chamber to achieve wet foam density of 0.28 g/ml ± 0.02.

Foam flow throughout the system was kept a flow rate of 6Kg/hr, and its temperature was regulated via a double-jacketed tubing to keep the gelatin solution above its transition temperature, to prevent it from freezing.

Midway throughout the streamline, enzyme solution at room temperature was introduced through an in-line valve, at a rate of 15 ml/min .

Static mixing elements inside the tubes (~2m long) allowed for adequate mixing of the mTG solution and the gelatin solution foam .

Mixed gelatin-mTG foam was ejected at the end of the line on top of a PEEK (poly ether ether ketone) flat board, serving as a mold. SurgicalMesh (SurgicalMesh, USA) surgical mesh prosthesis was pre-fixed to the PEEK mold, so that the foam applied on top of the mold covered it entirely .

Then foam was extruded with a Knife coater to the desired thickness (between 1000 and 1500 micrometers).

PEEK mold with the foam coated mesh was inserted to -80C refrigerator for freezing .

After 1.5 hours the mold was transferred to a lyophilizer (Virtis Genesis EL,SP Scientific) for a freeze-drying process (see Table 7).

**Table 7 - freeze drying program**

| Phase Name | Step # | Temperature, °C | Pressure, mTorr | Duration of step, minutes | Notes |
|---|---|---|---|---|---|
| Semi-Auto functions | - | -25 | 800 | - | This step is done prior to lyopilization (and before inserting the samples) in order to keep the shelves in low temperature |

| Freeze, Condenser and Evacuate | 1 | -20 | - | 1 | Vacuum start permit by condenser temperature - -60 °C Heat start permit by pressure - 900 mTorr |
|---|---|---|---|---|---|
| Primary Drying | 1 | -20 | 600 | 120 | Hold |
| | 2 | 0 | 600 | 60 | Rate (temperature) |
| | 3 | 0 | 300 | 120 | Hold (Step pressure drop) |
| | 4 | 0 | 150 | 60 | Rate |
| | 5 | 15 | 150 | 60 | Rate |
| | 6 | 15 | 50 | 60 | Rate |
| Secondary Drying | 1 | 15 | 50 | 2000 | Hold |
| Product temperature | - | 10 | - | - | |
| Storage temperature | - | 10 | - | - | |
| Storage Vacuum | | - | 100 | - | |

After freeze-drying, mesh articles coated by 1.0-1.5 mm foam containing a dry mixture of gelatin and mTG were obtained, by peeling the foam layer easily off the PEEK molds .

### Results

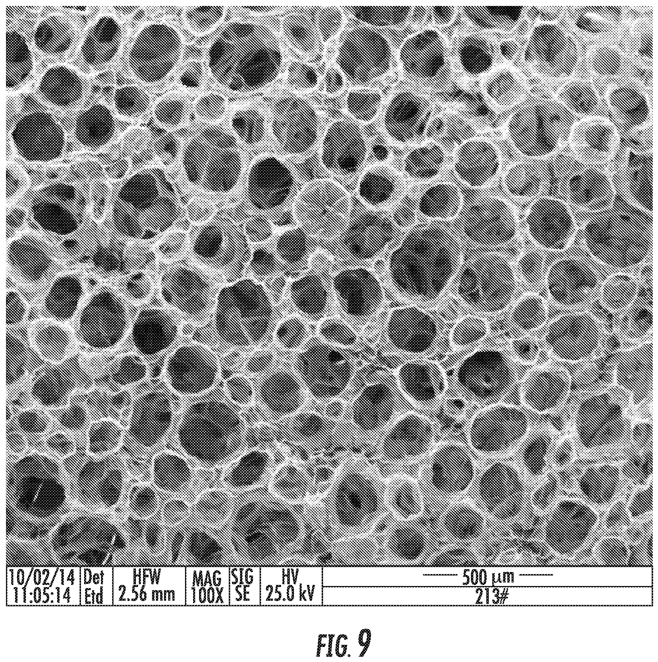

Figure 9 shows an SEM image of a Cross-section of a gelatin-mTG dry foam article, prepared by the process described in example 7;

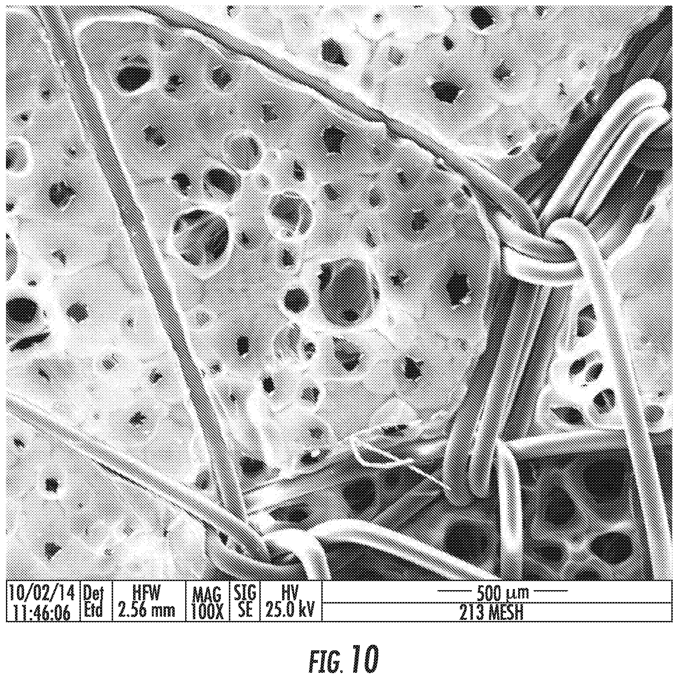

Figure 10 shows an SEM image of the adhesive surface of a gelatin-mTG dry foam article, prepared by the process described in example 7.

### Example 8 - Freeze-Drying of Non-Eutectic Gelatin In Water Solutions

### Methods:

Solutions of various concentration of gelatin in water were prepared, by mixing Bloom 275 gelatin particles (Gelita, USA) in DW at 50oC for 1 hour.

Solutions were acidified by titration of acetic acid 6M (BioLab) to pH=3.8.

In the final mixing stage, purified mTG stock solution at a concentration of 50 U/ml was added to the solution in a ratio of 80 U/g gelatin. Solution mixed thoroughly for 1 min. After addition of all materials, solutions contained 2.5% and 1.5% of gelatin in water (w/w).

Solutions were cooled down and kept over-night at 24.5°C, slightly above the freeze-thaw transition point of the gelatin solutions.

Vitamesh Blue (Proxy, Ireland) Hernia prosthesis 15 cm round shaped articles were fixed over a flat PEEK boards, serving as molds, which were pre-cooled to 5°C. Acidified gelatin-enzyme mixtures were applied in 1-1.5 mm layers on the flat PEEK board, covering the Vitamesh Blue articles, using a Knife coater.

The coated boards were inserted to a freezer at -80C for 1.5 hour, and after freezing, inserted to a lyophilizer (Virtis Genesis EL, SP Scientific) for the same freeze drying process as described in Table 7 above.

Dried foam-coated articles were peeled-off from the PEEK boards prior to inspection.

### Fixation strength in lap shear on collagen method:

Collagen sheets, cut to a size of 6x7 cm (WxL) were soaked in 0.9% saline until hydrated then heated to 36-37°C. Samples were prepared by cutting whole mesh based composition articles to pieces 5x7 cm (WxL) and covering 2 of the 7 cm with tape for gripping with the test machine. Mesh based composition samples were applied to preheated collagen sheets and allowed 4 minutes of curing in a 37°C incubator.

For testing at time zero: samples tested in lap shear using a model 3433 Instron universal testing machine immediately after the 4 minutes curing.

For testing after 24 hours of immersion in saline: Samples were placed in petri dishes filled with saline then left in a 37°C incubator for 24 hours. After 24 hours the samples were tested in lap shear using a model 3433 Instron universal testing machine.

### Results

Table 8 shows the results of the tests. As shown, a desirable gelatin content is at least 2% and preferably at least 2.5%.

| Gelatin concentration in solution | 1.5% w/w (according to example 8) | 2.5% w/w (according to example 8) |
|---|---|---|
| Production batch No. | GF-491 | GF-476 |
| Appearance | White foam coating over the polypropylene mesh | White foam coating over the polypropylene mesh |
| Feel (qualitative) | Very soft, delicate, low resistance to tearing | Soft and pliable |
| Thickness | 1.4-1.5 mm | 1.5-1.6 mm |
| Fixation strength to collagen after 24 hour immersion in 0.9% | 2.2 ± 0.3 * | 3.0 ± 0.2 |
| saline solution [N/cm] | | |

| | | |
|---|---|---|
| * crosslinked gelatin backing layer ~50 micrometers thick was attached in the back side of the sample for the sake of the measurement. | | |

### EXAMPLE 9 - Precise Positioning of the Mesh

Specific surgical mesh positioning in regard to the gelatin foam, according to at least some embodiments, may increase desirable properties, including with regard to tissue embedding. Surprisingly, the inventors have found that the position of the mesh within the foam coating may reduce the degree of tissue response to the prosthesis at initial stages of the product application on the tissue, without wishing to be limited by a single hypothesis.

### Method:

In the following examples no bonding layer was used, as the adhesive layer was directly applied over the pre-cured backing layer, bonding them together when the adhesive layer was dried.

Backing layer was prepared by coating PEEK flat boards:
Backing solution:1% HPMC K100 (Ashland), 1.7% HPMC K4 (Ashland), 0.9% PEG 400 (Merck)
Backing casting: The backing solution is poured on the PEEK liner and a knife coater (Doctor Blade) is used in order to adjust its height (1100µm).

The solution is dried at RT for at least 24 hours.

Gelatin-mTG foam (9% w/w gelatin in water and acetic acid, pH=3.8, mTG concentration - 80U/g gelatin) was prepared as described above under physical aeration with pressurized gas.

The following process modifications were implemented for the placement of Vitamesh Blue surgical mesh within the adhesive layer in various locations, shown in Table 9.

**Table 9 - modifications to test placement of mesh in regard to adhesive layer**

| Surgical mesh position within foam | d2+d3 (microns, estimated) * | d1 (microns, estimated) * | Preparation process |
|---|---|---|---|
| Middle (mesh | ~ 800 | ~ 400 | First wet gelatin-mTG foam layer extruded |
| fully surrounded by foam) Batch No. LM-147 | | | with a knife coater to 800 microns. Surgical mesh placed on top of the wet foam layer. Second foam layer, covering the surgical mesh, was extruded to 1200 microns. (see Figure 11A) |
| Surface (mesh slightly exposed to tissue) Batch No. LM-149 | ~ 1200 | ~ 0 | Foam layer extruded to 1400 microns. Surgical mesh placed on top of the wet foam layer. Tyvek (Dupont, USA) 1073B sheet was placed on top of the mesh, covering it. Knife coater at 1200 microns was conveyed over the Tyvek sheet, pressing the surgical mesh into the wet foam. Tyvek sheet was removed after freezing, before initiating the freeze-drying process. (see Figure 11B) |
| Middle/Surface alternating stripes pattern Batch No. LM-150 | ~800/ ~1100 | ~400/ ~100 ** | First wet gelatin-mTG foam layer extruded with a knife coater to 800 microns. Surgical mesh placed on top of the wet foam layer. Second foam layer, covering the surgical mesh, was extruded with a custom-made knife coater with 750/1200 microns alternating gap sections (each section 0.5 cm wide). |

| | | | |
|---|---|---|---|
| * corresponding to Figure 2A above ** design intended to be middle and surface alternating sections, so that mesh is exposed in the shallow sections. In practice, in these sections the mesh was fully covered by foam. | | | |

### Results:

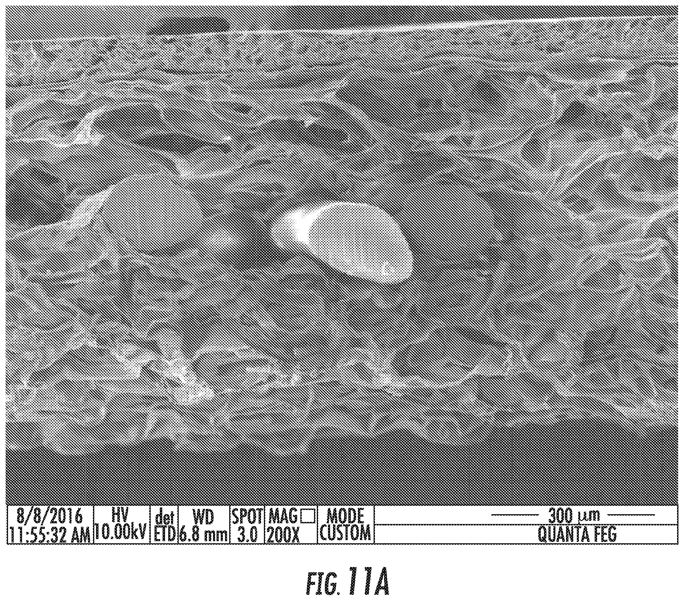

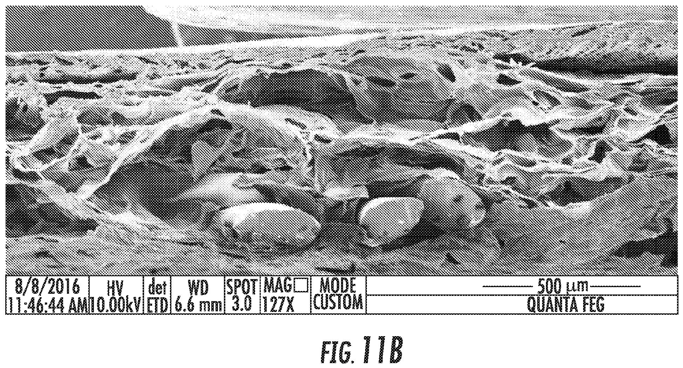

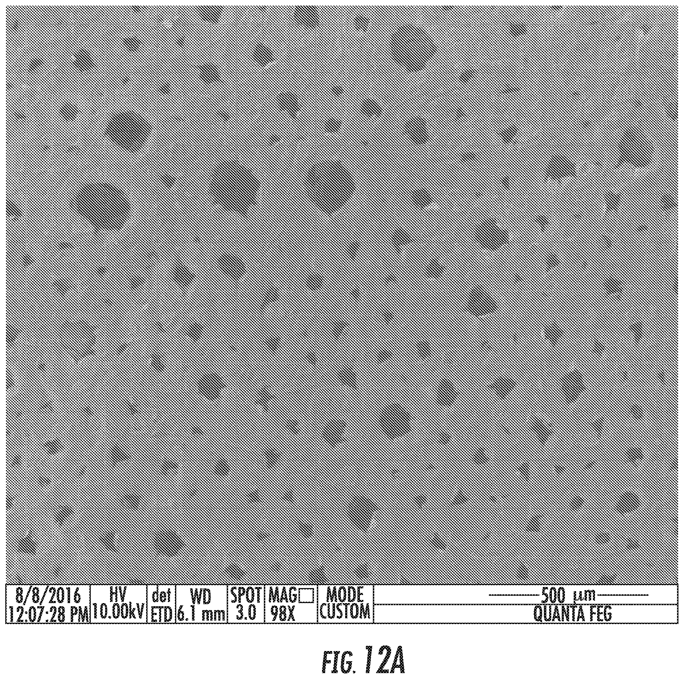

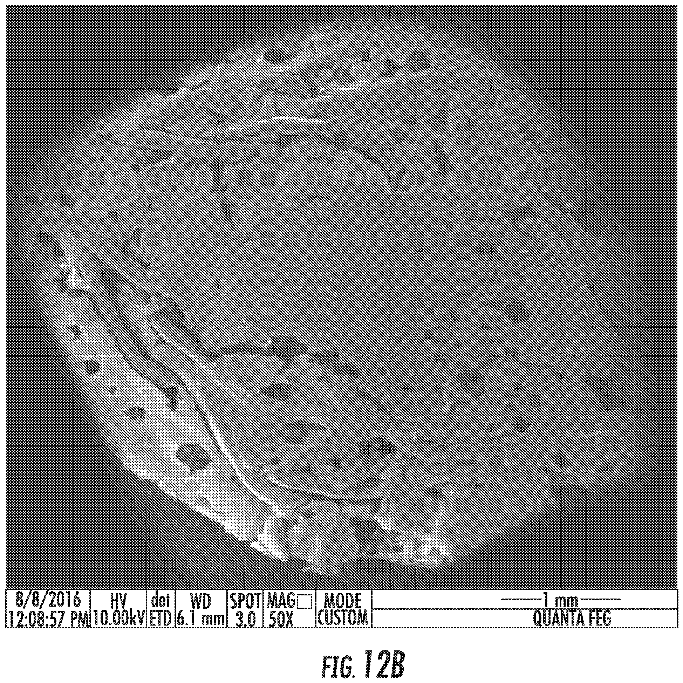

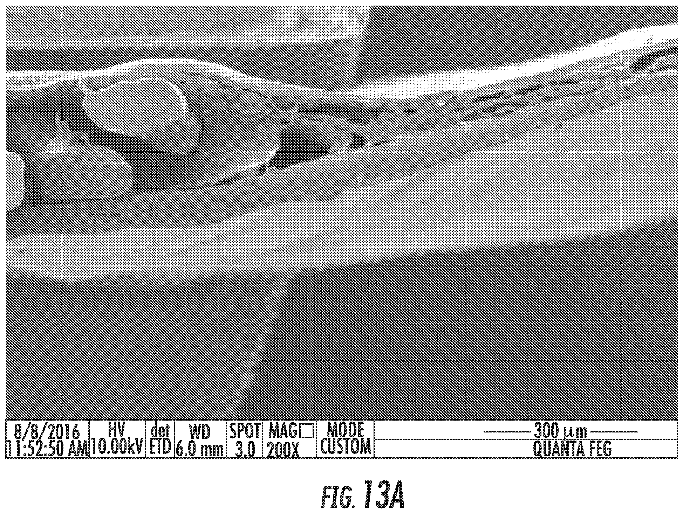

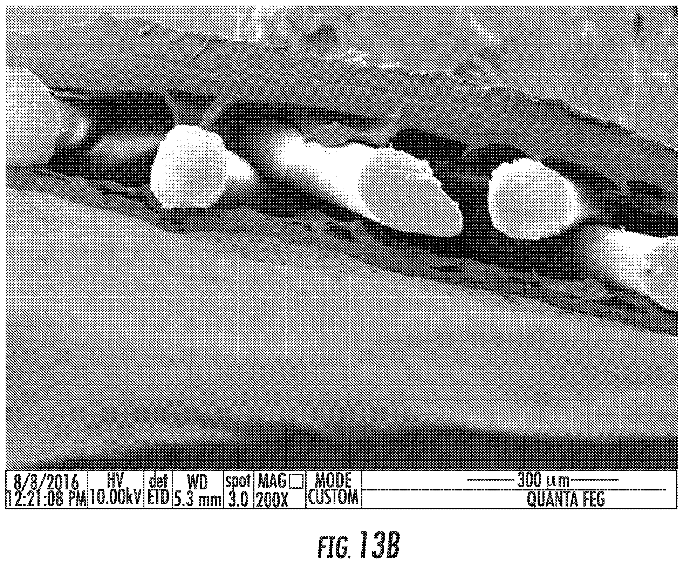

Figure 11A shows an SEM image of a cross-section of LM-147 article - Mesh in embedded in the middle of the foam. Figure 11B shows an SEM image of a cross-section of LM-149 article - Surgical mesh is located at the bottom (surface). Figure 12A shows an SEM image of the bottom surface of LM-147 article - Surgical mesh is not visible as it is fully covered by foam. Figure 12B shows an SEM image of the bottom surface of LM-149 article - Surgical mesh is located at the surface level, not fully covered. Figure 13A shows an SEM image of LM-149 after fixation on collagen as a tissue simulating substrate (collagen is the bottom layer). Figure 13B shows an SEM image of LM-147 after fixation on collagen as a tissue simulating substrate (collagen is the top layer).

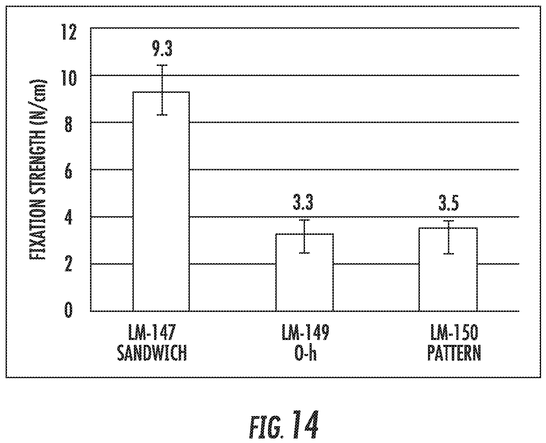

Figure 14 shows the fixation strength of the different models on collagen, as tested by the lap shear method, 4 minutes after fixation. The testing method was performed as described in the section on freeze-drying of non-eutectic gelatin in water solutions.

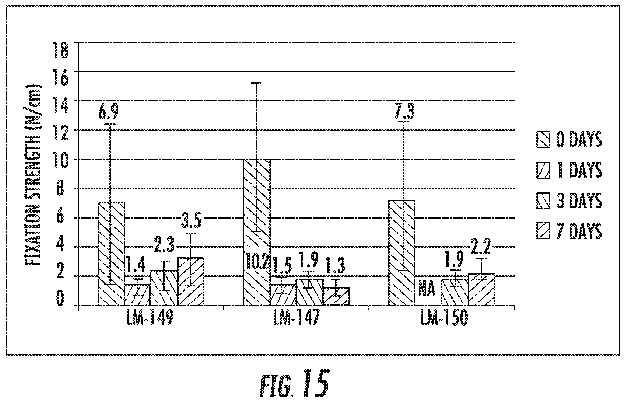

Figure 15 shows the fixation strength of the different models on swine peritoneum tissue, as tested by the lap shear method, different days after implantation (study AT-05-55). Fixation to tissue was tested ex-vivo. Testing method for 0, 3 and 7 days time-points: Explanted Mesh based compositions samples were cut to samples 4x7 cm (WxL), 1 cm of mesh was detached from the tissue on the 7 cm edge for gripping leaving a 4x6 cm effective testing area. Samples were tested in lap shear using an Instron universal testing machine while on one side only tissue was gripped and on the other only mesh was gripped. Day 1 samples were tested by opening the abdominal wall, separating the Mesh based composition for gripping (leaving 4x6 cm testing area) and pulling in lap shear using a force gauge.

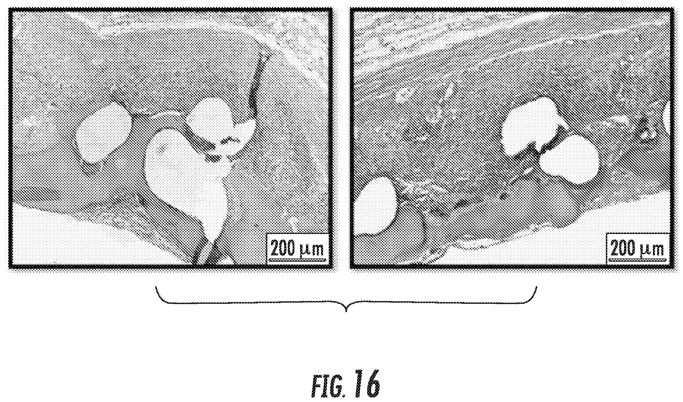

Figure 16 shows representative histopathology images of samples explanted after 7 days from swine. Left - LM-147, Right - LM-149. The original position of the mesh within the foam affects the amount of new tissue ingrowth surrounding the mesh (higher in LM-149 relative to LM-147).

### Example 10 - Adhesion layer - foam areal density

It was assumed that the porous gelatin structure of the foam is important for fast tissue integration into the surgical mesh. This is because the high effective surface area of the foam increases availability for enzymatic decomposition in the body, enabling fast degradation of the cross-linked gelatin (within days). As the crosslinked gelatin degrades, it makes room for the infiltration and proliferation of new tissue, encapsulating the surgical mesh fibers and fixating it firmly to place. On the other hand, increasing the foam load per area can contribute to the strength of the crosslinked gelatin gel matrix, which is the main load bearing fixation element in the few days after implantation. Therefore, the optimum between fast tissue integration and initial fixation strength of the gel matrix was sought for.

### Method:

Note - in the following examples no bonding layer was used, as the adhesive layer was directly applied over the pre-cured backing layer, bonding them together when the adhesive layer was dried.

Gelatin-mTG foam (9% w/w gelatin in water and acetic acid, pH=3.8, mTG concentration - 80U/g gelatin) was prepared as described in the above section on foaming with gas .

The following process modifications were implemented, as shown in Table 10.

**Table 10 - Process Modifications**

| Surgical mesh position within foam | d2+d3 (mm)* | d1 (microns) * | Preparation process |
|---|---|---|---|
| Surface (mesh slightly exposed to tissue) | ~ 0.9 -1.2 | ~ 0 | Foam layer extruded to 1400 microns. Surgical mesh placed on top of the wet foam layer. Tyvek 1073B sheet (Dupont, USA) was placed on top of the mesh, covering it. Knife coater at 1200 microns was conveyed over the Tyvek sheet, pressing the surgical mesh into the wet foam. Tyvek sheet was removed after freezing, before initiating the freeze-drying process. |
| Surface (mesh slightly exposed to tissue) | ~ 1.3 - 1.6 | ~ 0 | Foam layer extruded to 2000 microns. Surgical mesh placed on top of the wet foam layer. Tyvek 1073B sheet (Dupont, USA) was placed on top of the mesh, covering it. Knife coater at 1200 microns was conveyed over the Tyvek sheet, pressing the surgical mesh into the wet foam. Tyvek sheet was removed after freezing, before initiating the freeze-drying process. |

| | | | |
|---|---|---|---|
| * corresponding to Figure 2A above | | | |

### Results

Table 11 shows the results of the different foam depth applications, with a greater depth in the left column (2000 microns) and a lower depth in the right column (1400 microns).

**Table 11 - effect of foam depth**

| | | |
|---|---|---|
| Initial foam extrusion gap (microns) | 2000 | 1400 |
| Production batch No. | LM-272 | LM-275 |
| Thickness | 1.35 ± 0.05 mm | 1.0 ± 0.1 mm |
| Areal density of gelatin-mTG dry foam (mg/cm2) | 3.4 ± 0.1 | 1.8 ± 0.2 |
| Fixation strength to collagen after 24 hour immersion in 0.9% saline solution [N/cm] | 1.6 ± 0.3 | 1.2 ± 0.1 |

### Example 11 - Backing alternatives and adhesion barrier properties of crosslinked gelatin

The product contains a surgical mesh embedded within a water-activated adhesive coating. As described in the provisional application, it also contains a backing layer that prevents from the product to be sticky towards its back side (i.e. to the gloves, surgical tools, etc. with which the surgeon applies the product).

Another important feature, which is required in intraperitoneal mesh implantations, is the prevention of adhesions of the implant to visceral organs, e.g. bowels, liver, spleen and bladder. This property is commonly termed "adhesion barrier " and is known in prior art.

### Two non-adhesive backing versions were examined throughout development:

The first backing version was HPMC based (hydroxy propyl methyl cellulose K4M 22.3%wt, hydroxy propyl methyl cellulose K100M 44.6%wt, PEG 400 33%wt). The HPMC backing serves only the purpose of prevention of adhesion of the product to surgical tools and wet gloves. It is water-soluble, therefore it dissolves in the fluids existing inside the abdominal cavity within a few hours after implantation. The adhesion barrier properties are coming from the remaining gelatin layer, which covers the surgical mesh completely and has already been fully crosslinked while the HPMC based backing had dissolved away.

The second backing version was crosslinked gelatin based (Gelatin 70%, mTG - 5 U/g gelatin, PEG 400 30%wt). The crosslinked gelatin backing fuses with the in-situ crosslinking adhesive gelatin-mTG layer to become a continuous crosslinked gelatin matrix. During application on tissue it is non-adhesive since it is already crosslinked, therefore preventing sticking in its back side, and it also serves as additional reinforcement to the adhesive, increasing the overall gel mass. Being non-adhesive, it is also an adhesion barrier to visceral organs.

### Methods

HPMC backing (GF-199):
Solution: 1% HPMC K100 (Ashland), 0.3% PEG400 (Aldrich), autoclaved.
Process: Teflon coated trays (12cmX17cm and 9.5cmX9.5cm) were filled with 60 ml and 26 ml, respectfully, of the solution and allowed to dry at RT.

Heat cross linked Gelatin backing (GF-200):
Solution: 1% gelatin 225 type A (Gelita), filtered through 0.2µm filter.
Process: ~60 ml of the solution were cast into 9.5cmX9.5cm Teflon coated trays. The solution was allowed to dry at RT, then were incubated in an oven at 160°c for 2-4 hours.

Enzyme cross linked gelatin backing (LM-274):
Solution: 5% gelatin (Gelita), 2.1% PEG 400 (Merck) + 5u purified mTG enzyme (TP1701) /g gelatin (total in sol.)
Process: The solution is prepared without the enzyme. Before application of the solution on the liner, the enzyme is added, mixed and then the solution applied and set to 1000µm height using a gap controller (Doctor Blade). The backing is allowed to dry at RT (while cross linking by the enzyme).

### Results

Fixation strength to collagen after 24 hour immersion in 0.9% saline solution was tested for two models: LM-274 (crosslinked gelatin based backing layer) and LM-216 (HPMC based backing layer). The results are as follows are shown in Table 12.

**Table 12 - effect of crosslinked gelatin vs HPMC backing**

| | | |
|---|---|---|
| Mesh based composition production batch number | LM-274 | LM-216 |
| Backing layer | Crosslinked gelatin | HPMC |
| Fixation strength to collagen after 24 hours immersion in 0.9% saline solution (N/cm) | 1.9 ± 0.3 | 1.0 ± 0.05 |

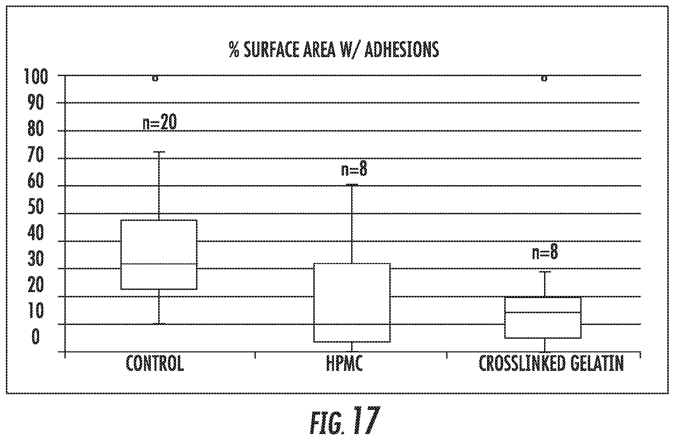

Figure 17 shows a boxplot representing % surface area of Control mesh (bare Surgical Mesh) and Mesh based composition^{™} (Surgical mesh covered with adhesive) groups GF-199 (Crosslinked gelatin), GF-200 (HPMC) covered by adhesions, 14 days after implantation. The study was performed with intraperitoneal implantation of the articles in swine. This Example demonstrates that either of HPMC or cross-linked gelatin may optionally be used as backing.

### Example 12 - Gelatin - Alginate mesh based composition

### Preparation of gelatin/alginate solution

300 g purified water was heated to approximately 40°C. 8.35 g gelatin powder (Gelita, Type A gelatin, Bloom 275) was added to the heated water and mixed with a magnetic stirrer until homogenous.

98.35 g purified water was heated to approximately 40°C. 1.68 g low viscosity sodium alginate (Sigma Aldrich, cat# A0682) was added to the heated water and mixed with a magnetic stirrer until homogenous.

The alginate solution was added to the gelatin solution and mixed. As phase separation occurs when alginate and gelatin solutions are mixed, 3.4 ml 6M acetic acid (BioLab, Israel) and 5.7 g NaCl (Merck) were until mixture was at a pH of 3.8 and solution was clear.

74.72 g purified water was added to complete to a weight of 490.5 g.

### Preparation of gelatin/alginate coated surgical mesh (Batch #GF-502)

Purified mTG enzyme stock solution at a concentration of 50 U/ml was added to the acidified gelatin -alginate solution in a ratio of 80 U/g gelatin. Solution mixed thoroughly for 1 min.

Vitamesh Blue (Proxy, Ireland) Hernia prosthesis 15 cm round shaped articles were fixed over a flat PEEK boards, serving as molds, which were pre-cooled to 5°C. Acidified gelatin -alginate -enzyme mixtures were applied in 1-1.5 mm layers on the flat PEEK board, covering the Vitamesh Blue articles, using a Knife coater.

The coated boards were inserted to a freezer at -80C for 1.5 hour, and after freezing, inserted to a lyophilizer (Virtis Genesis EL,SP Scientific) for the same freeze drying process as described in Table 7 above.

Dried foam-coated articles were peeled-off from the PEEK boards prior to inspection.

Testing fixation strength 1 day post application of gelatin/alginate LifeMesh (GF-502) with a calcium source.

LifeMesh were cut to samples 5x7 cm (WxL) and 2 cm of the Length were covered with tape, allowing for a place to grip the sample with the testing machine. Collagen sheets were cut to pieces 6x7 cm (WxL) and preheated to 36-37°C, while covered with saline soaked gauze. LifeMesh samples were applied to collagen and allowed 4 minutes to cure in a 37°C incubator. After curing, samples were submerged in 0.9% saline / 0.9% saline containing a calcium source (CaCl₂). Samples were kept in 37°C for 24 hours then tested in lap shear using a model 3433 Instron universal testing machine.

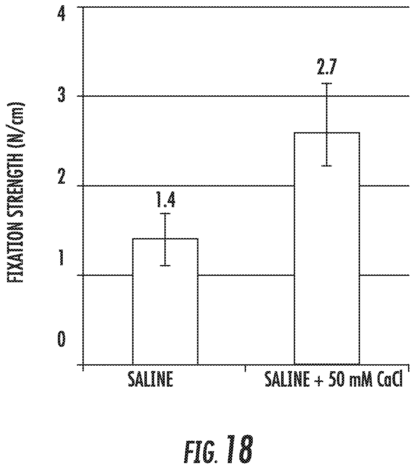

Figure 18 shows the fixation strength of Gelatin - Alginate mesh based composition (batch No. GF-502) on collagen, as tested by the lap shear method, 24 hours after fixation. GF-502 articles were immersed for 24 hours post application on collagen in either 0.9% saline or 0.9% saline with addition of 50mM CaCl2.

### Example 13 - Gelatin - Chitosan mesh based compositions

Three mixtures of gelatin (Gelita, Type A gelatin, Bloom 275) and low MW chitosan (Aldrich cat# 448869) were prepared (see table 13), each with total solids content of 2% w/w, and total weight of 300 g each.

The mixtures were brought to pH 3.8 with acetic acid, in order to allow dissolution of the chitosan. mTG was added to a final ratio of 80 U per gram gelatin.

**Table 13 - Gelatin - Chitosan mesh based compositions :**

| Batch no. | % chitosan | Chitosan (gr) | Gelatin (gr) | Acetic acid (ml) |
|---|---|---|---|---|
| GF512 | 5 | 0.3 | 5.7 | 5.4 |
| GF511 | 10 | 0.6 | 5.4 | 5.7 |
| GF510 | 20 | 1.2 | 4.8 | 9 |

The solution was cooled down to 16°C, casted on PEEK plates at a layer thickness of 250µm using a doctor blade. Surgical mesh was placed on the liquid layer and the composition was cooled down at 4°C. Another layer was placed on top of the surgical mesh at a thickness of 1700 µm using a doctor blade. The coated mesh was then frozen at -80°C and lyophilized.

The resulting dry coated meshes were tested for fixation strength to collagen as described in Example 12.

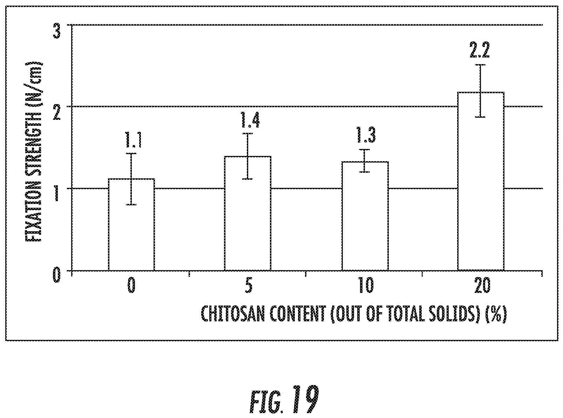

Figure 19 shows the fixation strength of Gelatin- Chitosan mesh based compositions (batches No. GF-510, GF-511, GF-512) on collagen, as tested by the lap shear method, 24 hours after fixation. The articles were immersed for 24 hours post application on collagen in 0.9% saline.

## Claims

1. A mesh-based composition comprising a mesh and a coating, wherein said coating is a foamed composition comprising one or more cross-linkable proteins or polypeptides and one or more cross-linking materials, wherein at least a portion of said mesh is coated with said coating such that the mesh-based composition comprises two sections, a mesh-adhesive section which is composed of a mesh enclosed within said coating and a coating-only section which contains said coating alone, without mesh within it, wherein the coating-only section surrounds the mesh-adhesive section, and wherein the coated mesh is self-adhering, requires no additional fixation, and is capable of minimizing tissue adhesions upon application.

2. The composition of claim 1, wherein the cross-linkable protein or polypeptide comprises gelatin, wherein the gelatin is foamed, wherein the gelatin foam is in a density range of 1 to 100 mg/cm3 and preferably in the range of 1 to 50 mg/cm3.

3. The composition of claim 2, wherein said foamed gelatin comprises dried or lyophilized foamed gelatin.

4. The composition of any of the above claims, further comprising a non-sticky, protective backing.

5. The composition of claim 4, wherein the backing comprises one or more cellulose ether derivatives selected from the group consisting of: HPMC (hydroxypropyl methylcellulose), HPC (hydroxypropyl cellulose), HEC (hydroxyethyl cellulose) and EC (ethyl cellulose).

6. The composition of any of the above claims, wherein said coating comprises said foamed composition and wherein said foamed composition is foamed according to a process selected from the group consisting of a batch mixing process, a continuous mixing process, a chemical foaming process, a Venturi foaming process and freeze drying.

7. The composition of any of the above claims, further comprising chitosan.

8. The composition of any of the above claims, further comprising calcium and a calcium crosslinkable alginate matrix.

9. The composition of any of the above claims, wherein said coating extends beyond said mesh, such that a total area of said mesh-based composition is larger than a total area of said mesh.

10. The composition of any of the above claims, wherein said one or more cross-linking materials comprise transglutaminase.

11. The composition of any of the above claims, wherein said one or more cross- linking materials comprise transglutaminase and wherein the transglutaminase comprises a plant, recombinant animal, and/or microbe derived transglutaminase other than blood derived Factor XIII, or wherein said transglutaminase comprises microbial transglutaminase.

12. The composition of any of the above claims, wherein said mesh-based composition features a backing layer comprising a resorbable material.

13. The composition of any of the above claims, structured as a rectangle or rounded shape, wherein a diameter or distance from edge to edge is in a range of from 0.5 cm to 60 cm, optionally wherein an area of the total mesh-based composition ranges from 0.5 cm2 to 3000 cm2 for round shaped mesh-based compositions, and from 0.5 cm2 to 4000 cm2 for square shaped mesh-based compositions.

14. The composition of claim 13, wherein an area of the mesh in cm squared ranges from 0.5 cm2 to 3600 cm2.

## Patentansprüche

1. Netzbasierte Zusammensetzung, umfassend ein Netz und eine Beschichtung, wobei die Beschichtung eine geschäumte Zusammensetzung ist, umfassend ein oder mehrere vernetzbare Proteine oder Polypeptide und ein oder mehrere vernetzende Materialien, wobei mindestens ein Abschnitt des Netzes mit der Beschichtung beschichtet ist, sodass die netzbasierte Zusammensetzung zwei Sektionen umfasst, eine netzhaftende Sektion, die aus einem in der Beschichtung eingeschlossenen Netz besteht, und eine beschichtungsreine Sektion, die nur die Beschichtung enthält, ohne Netz darin, wobei die beschichtungsreine Sektion die netzhaftende Sektion umgibt und wobei das beschichtete Netz selbsthaftend ist, keine zusätzliche Fixierung erfordert und in der Lage ist, Gewebeadhäsionen bei Anwendung zu minimieren.

2. Zusammensetzung nach Anspruch 1, wobei das vernetzbare Protein oder Polypeptid Gelatine umfasst, wobei die Gelatine geschäumt ist, wobei der Gelatineschaum in einem Dichtebereich von 1 bis 100 mg/cm3 und vorzugsweise in einem Bereich von 1 bis 50 mg/cm3 liegt.

3. Zusammensetzung nach Anspruch 2, wobei die geschäumte Gelatine getrocknete oder lyophilisierte geschäumte Gelatine umfasst.

4. Zusammensetzung nach einem der vorstehenden Ansprüche, ferner umfassend eine nichtklebrige Schutzunterlage.

5. Zusammensetzung nach Anspruch 4, wobei die Unterlage ein oder mehrere Celluloseetherderivate umfasst, ausgewählt aus der Gruppe, bestehend aus: HPMC (Hydroxypropylmethylcellulose), HPC (Hydroxypropylcellulose), HEC (Hydroxyethylcellulose) und EC (Ethylcellulose).

6. Zusammensetzung nach einem der vorstehenden Ansprüche, wobei die Beschichtung die geschäumte Zusammensetzung umfasst und wobei die geschäumte Zusammensetzung gemäß einem Prozess ausgewählt aus der Gruppe, bestehend aus einem Batch-Mischprozess, einem kontinuierlichen Mischprozess, einem chemischen Schäumungsprozess, einem Venturi-Schäumungsprozess und Gefriertrocknung geschäumt wird.

7. Zusammensetzung nach einem der vorstehenden Ansprüche, ferner umfassend Chitosan.

8. Zusammensetzung nach einem der vorstehenden Ansprüche, ferner umfassend Calcium und eine mit Calcium vernetzbare Alginatmatrix.

9. Zusammensetzung nach einem der vorstehenden Ansprüche, wobei die Beschichtung über das Netz hinausreicht, sodass die Gesamtfläche der netzbasierten Zusammensetzung größer ist als die Gesamtfläche des Netzes.

10. Zusammensetzung nach einem der vorstehenden Ansprüche, wobei das eine oder die mehreren vernetzenden Materialien Transglutaminase umfassen.

11. Zusammensetzung nach einem der vorstehenden Ansprüche, wobei das eine oder die mehreren vernetzenden Materialien Transglutaminase umfassen und wobei die Transglutaminase eine von einer Pflanze, einem rekombinanten Tier und/oder einem Mikroorganismus abgeleitete Transglutaminase umfasst, die sich von dem aus Blut gewonnenen Faktor XIII unterscheidet, oder wobei die Transglutaminase mikrobielle Transglutaminase umfasst.

12. Zusammensetzung nach einem der vorstehenden Ansprüche, wobei die netzbasierte Zusammensetzung eine Unterlage aufweist, die ein resorbierbares Material umfasst.

13. Zusammensetzung nach einem der vorstehenden Ansprüche, die als Rechteck oder abgerundete Form strukturiert ist, wobei ein Durchmesser oder eine Entfernung von Kante zu Kante in einem Bereich von 0,5 cm bis 60 cm liegt, optional wobei eine Fläche der gesamten netzbasierten Zusammensetzung von 0,5 cm2 bis 3000 cm2 für runde geformte netzbasierte Zusammensetzungen und von 0,5 cm2 bis 4000 cm2 für quadratische geformte netzbasierte basierende Zusammensetzungen reicht.

14. Zusammensetzung von Anspruch 13, wobei die Fläche des Netzes in Quadratzentimetern von 0,5 cm2 bis 3600 cm2 reicht.

## Revendications

1. Composition à base d'une structure maillée comprenant une maille et un revêtement, dans laquelle ledit revêtement est une composition expansée comprenant une ou plusieurs protéines ou polypeptides réticulables et un ou plusieurs matériaux de réticulation, dans laquelle au moins une partie de ladite maille est revêtue dudit revêtement de telle sorte que la composition à base d'une structure maillée comprend deux sections, une section adhésive de maille qui est composée d'une maille enfermée dans ledit revêtement et une section de revêtement uniquement qui contient ledit revêtement seul, sans maille à l'intérieur, dans laquelle la section de revêtement uniquement entoure la section adhésive de maille, et dans laquelle la maille revêtue est auto-adhésive, ne nécessite aucune fixation supplémentaire et est capable de minimiser des adhérences tissulaires lors de l'application.

2. Composition selon la revendication 1, dans laquelle la protéine ou le polypeptide réticulable comprend de la gélatine, dans laquelle la gélatine est expansée, dans laquelle la mousse de gélatine est dans une plage de densité de 1 à 100 mg/cm3 et de préférence dans la plage de 1 à 50 mg/cm3.

3. Composition selon la revendication 2, dans laquelle ladite gélatine moussée comprend de la gélatine moussée séchée ou lyophilisée.

4. Composition selon l'une quelconque des revendications précédentes, comprenant également un support protecteur non collant.

5. Composition selon la revendication 4, dans laquelle le support comprend un ou plusieurs dérivés d'éther de cellulose choisis dans le groupe constitué par : HPMC (hydroxypropylméthylcellulose), HPC (hydroxypropylcellulose), HEC (hydroxyéthylcellulose) et EC (éthylcellulose).

6. Composition selon l'une quelconque des revendications précédentes, dans laquelle ledit revêtement comprend ladite composition expansée et dans laquelle ladite composition expansée est expansée selon un procédé choisi dans le groupe constitué par un procédé de mélange par lots, un procédé de mélange continu, un procédé de moussage chimique, un procédé de moussage Venturi et une lyophilisation.

7. Composition selon l'une quelconque des revendications précédentes, comprenant également du chitosane.

8. Composition selon l'une quelconque des revendications précédentes, comprenant également du calcium et une matrice d'alginate réticulable de calcium.

9. Composition selon l'une quelconque des revendications précédentes, dans laquelle ledit revêtement se prolonge au-delà de ladite maille, de telle sorte qu'une surface totale de ladite composition à base d'une structure maillée est plus grande qu'une surface totale de ladite maille.

10. Composition selon l'une quelconque des revendications précédentes, dans laquelle lesdits un ou plusieurs matériaux de réticulation comprennent de la transglutaminase.

11. Composition selon l'une quelconque des revendications précédentes, dans laquelle lesdits un ou plusieurs matériaux de réticulation comprennent de la transglutaminase et dans laquelle la transglutaminase comprend une transglutaminase dérivée d'une plante, d'un animal recombinant et/ou d'un microbe autre que le facteur XIII dérivé du sang, ou dans laquelle ladite transglutaminase comprend une transglutaminase microbienne.

12. Composition selon l'une quelconque des revendications précédentes, dans laquelle ladite composition à base d'une structure maillée présente une couche de support comprenant un matériau résorbable.

13. Composition selon l'une quelconque des revendications précédentes, structurée sous la forme d'un rectangle ou d'une forme arrondie, dans laquelle un diamètre ou une distance d'un bord à l'autre est compris dans une plage de 0,5 cm à 60 cm, éventuellement dans laquelle une surface de la composition totale à base d'une structure maillée est comprise dans une plage de 0,5 cm2 à 3 000 cm2 pour les compositions à base d'une structure maillée de forme ronde, et de 0,5 cm2 à 4 000 cm2 pour des compositions à base d'une structure maillée de forme carrée.

14. Composition selon la revendication 13, dans laquelle une surface de la maille en cm carré varie de 0,5 cm2 à 3 600 cm2.
